(19) Europäisches Patentamt
European Patent Office
Office européen des brevets

(11) Veröffentlichungsnummer: **0 216 165 B1**

# (12) EUROPÄISCHE PATENTSCHRIFT

(45) Veröffentlichungstag der Patentschrift: **23.11.89**

(21) Anmeldenummer: **86111728.1**

(22) Anmeldetag: **25.08.86**

(51) Int. Cl.⁴: **C07D 487/04,** A61K 31/40, C07D 405/12, C07D 409/12, C07D 403/12, C07D 417/12, C07D 413/12, C07D 401/12, C07D 209/30 // (C07D487/00, 209:00, 209:00)

## (54) Neue Benzodipyrrole, Verfahren zu ihrer Herstellung und diese Verbindungen enthaltende Arzneimittel.

(30) Priorität: **29.08.85 DE 3530825**

(43) Veröffentlichungstag der Anmeldung: **01.04.87 Patentblatt 87/14**

(45) Bekanntmachung des Hinweises auf die Patenterteilung: **23.11.89 Patentblatt 89/47**

(84) Benannte Vertragsstaaten: **AT BE CH DE FR GB IT LI LU NL SE**

(56) Entgegenhaltungen:
**DE-A-1 795 330**
**DE-A-2 710 464**
**DE-A-3 446 417**
**DE-C-648 639**
**US-A-3 505 353**

**ARCHIV DER PHARMAZIE, vol. 304, 1971. H. AUTERHOFF et al.: "Reaktionen des Nicotyrins mit 4-Dimethylaminobenzaldehyd", Seiten 288-296**

**Die Akte enthält technische Angaben, die nach dem Eingang der Anmeldung eingereicht wurden und die nicht in dieser Patentschrift enthalten sind.**

(73) Patentinhaber: **BOEHRINGER MANNHEIM GMBH, Sandhofer Strasse 116, D-6800 Mannheim 31(DE)**

(72) Erfinder: **Mertens, Alfred, Dr. rer. nat., Beethovenstrasse 20, D-6905 Schriesheim(DE)**
Erfinder: **von der Saal, Wolfgang, Dr. rer. nat., Neuer Burgweg 3, D-6940 Weinheim(DE)**
Erfinder: **Berger, Herbert, Dr. phil., Völklinger Strasse 16, D-6800 Mannheim 31(DE)**
Erfinder: **Müller-Beckmann, Bernd, Dr. med. vet., Hochgewanne 46, D-6718 Grünstadt(DE)**
Erfinder: **Strein, Klaus, Prof., Eichenstrasse 45, D-6944 Hemsbach(DE)**

EP 0 216 165 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue Benzodipyrrole der allgemeinen Formel I

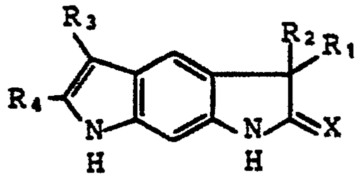

(I),

in welcher
$R_1$ ein Wasserstoffatom, eine Alkyl-, Alkenyl- oder eine Cycloalkylgruppe bedeutet,
$R_2$ ein Wasserstoffatom, eine Alkyl-, Alkenyl- oder Cyangruppe,
eine durch eine Hydroxy-, Alkyl-, Alkoxy-, Amino-, Alkylamino-, Dialkylamino- oder Hydrazinogruppe substituierte Carbonylgruppe oder mit $R_1$ zusammen eine Cycloalkylengruppe darstellt oder $R_1$ und $R_2$ zusammen eine Alkyliden- oder Cycloalkylidengruppe bilden,
$R_3$ ein Wasserstoffatom, eine Alkyl-, Alkenyl-, Cycloalkyl-, Cycloalkenyl-, Cyan-, Alkylcarbonyl-, Alkoxycarbonyl-, Carboxy, Aminocarbonyl-, Alkylaminocarbonyl-, Dialkylaminocarbonyl- oder Arylgruppe bedeutet,
$R_4$ ein Wasserstoffatom, eine Alkyl-, Trihalogenmethyl-, Cycloalkyl-, Hydroxy-, Cyan-, Carboxy-, Alkoxycarbonyl-, Alkylcarbonyl-, Aminocarbonyl-, Alkylaminocarbonyl- oder Dialkylaminocarbonylgruppe bedeutet oder
einen heterocyclischen Fuenfring mit 1-4 Heteroatomen oder einen heterocyclischen Sechsring mit 1-5 Heteroatomen darstellt, wobei die Heteroatome der vorgenannten Fuenf- und Sechsringe gleich oder verschieden sein koennen und Stickstoff, Sauerstoff oder Schwefel bedeuten und gegebenenfalls an einem oder mehreren Stickstoffatomen ein Sauerstoffatom tragen koennen, und die vorgenannten Fuenf- und Sechsringe gegebenenfalls durch eine oder mehrere Alkyl-, Alkoxy-, Alkylmercapto-, Hydroxy-, Nitro-, Amino-, Halogen- oder Cyangruppen substituiert sein koennen,
oder einen Phenylring der allgemeinen Formel II darstellt,

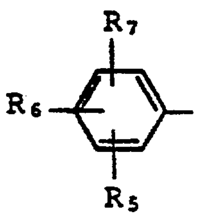

(II),

wobei $R_5$, $R_6$, $R_7$ gleich oder verschieden sein koennen und jeweils Wasserstoff, eine Alkansulfonyloxy-, Trifluormethansulfonyloxy-, Alkansulfonylamino-, Trifluormethansulfonylamino-, N-Alkyl-alkansulfonylamino-, N-Alkyltrifluormethansulfonylamino-, Alkylsulfenylmethyl-, Alkylsulfinylmethyl- oder Alkylsulfonylmethylgruppe, eine durch eine Hydroxy-, Alkoxy-, Amino-, Alkylamino- oder Dialkylaminogruppe substituierte Carbonylgruppe, eine durch eine Amino-, Alkylamino-, Dialkylamino- oder cyclische Iminogruppe substituierte Sulfonylgruppe, wobei eine Methylengruppe in 4-Stellung durch ein Schwefel- oder Sauerstoffatom ersetzt sein kann, eine Alkylcarbonylamino-, Aminocarbonylamino- oder Alkylaminocarbonylaminogruppe, eine Alkylmercapto-, Alkylsulfinyl- oder
Alkylsulfonylgruppe, eine Nitro-, Halogen-, Amino-, Hydroxy-, Alkyl-, Alkoxy-, Alkenyloxy-, Alkinyloxy-, Cyanalkyloxy-, Carboxyalkyloxy-, Alkoxycarbonylalkyloxy-, Dialkylamino-, 1-Imidazolyl-, Trifluormethyl- oder Cyangruppe sein koennen,
X ein Sauerstoff- oder Schwefelatom darstellt,
deren Tautomere und deren physiologisch vertraegliche Salze anorganischer und organischer Saeuren und Verfahren zu ihrer Herstellung, sowie diese Verbindungen enthaltende Arzneimittel.

Da die Verbindungen der allgemeinen Formel I fuer den Fall, daß $R_1$ nicht gleich $R_2$ ist, ein asymmetrisches Kohlenstoffatom besitzen, sind auch Gegenstand der Erfindung die optisch aktiven Formen und racemischen Gemische dieser Verbindungen.

Fuer die Verbindungen der allgemeinen Formel I, in der $R_4$ die Hydroxygruppe bedeutet, sind auch die tautomeren Formen 1 a und 1 b Gegenstand der Erfindung.

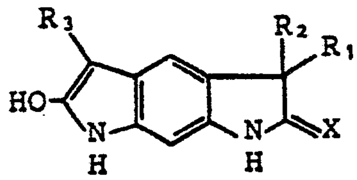

1 a

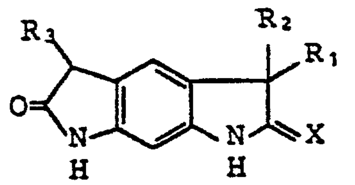

1 b

Diese neuen Verbindungen der vorliegenden Erfindung weisen wertvolle pharmakologische Eigenschaften auf, insbesondere steigern sie die Herzkraft und/oder wirken blutdrucksenkend und/oder beeinflussen die Thrombozytenfunktion und verbesseren die Mikrozirkulation.

In der allgemeinen Formel I koennen die Substituenten $R_1$ und $R_2$ gleich oder verschieden sein und Wasserstoff, eine Alkyl-, Cycloalkyl- oder Alkenylgruppe, eine Cyangruppe oder eine durch eine Hydroxy, Alkyl-, Alkoxy-, Amino-, Alkylamino-, Dialkylamino- oder Hydrazinogruppe substituierte Carbonylgruppe darstellen, wobei jeder der vorgenannten Alkyl- und Alkenylteile geradkettig oder verzweigt sein kann und 1-6 bzw. 2-6 Kohlenstoffatome und der vorgenannte Cycloalkylteil 3-7 Kohlenstoffatome enthalten kann.

Bevorzugt in diesem Sinne sind fuer $R_1$ und $R_2$ Wasserstoff, die Methyl-, Ethyl-, Isopropyl-, 3-Pentyl-, Allyl-, Cyclopentyl-, Cyclohexyl-, Cyan-, Acetyl-, Propionyl-, Methoxycarbonyl-, Ethoxycarbonyl-, Aminocarbonyl-, Methylaminocarbonyl-, Dimethylaminocarbonyl- und Hydrazinocarbonylgruppe.

$R_1$ und $R_2$ koennen zusammen mit dem C-Atom, an das sie gebunden sind, auch einen Cycloalkylring mit 3-7 Kohlenstoffatomen bilden, vorzugsweise handelt es sich dabei um die Spirocyclopropyl-, Spirocyclobutyl-, Spirocyclopentyl- und Spirocyclohexylgruppe.

$R_1$ und $R_2$ koennen zusammen auch eine Alkyliden- oder Cycloalkylidengruppe bilden, vorzugsweise handelt es sich dabei um die Isopropylidengruppe.

Bedeutet $R_3$ ein Wasserstoffatom, eine Alkyl-, Alkenyl-, Cycloalkyl- oder Cycloalkenylgruppe, so sind in diesem Sinne bevorzugt das Wasserstoffatom, geradkettige oder verzweigte Alkyl- oder Alkenylgruppen mit 1-7 bzw. 2-7 Kohlenstoffatomen und Cycloalkyl- oder Cycloalkenylgruppen mit 3-7 Kohlenstoffatomen. Vorzugsweise handelt essich um das Wasserstoffatom, die Methyl-, Ethyl-, Propyl-, Isopropyl-, n-Butyl-, Isobutyl-, 3-Pentyl-, Allyl-, 2-Butenyl-, Cyclopentyl-, Cyclohexyl-, Cyclopentenyl- und Cyclohexylgruppe.

Bedeutet $R_3$ eine Cyan-, Alkylcarbonyl-, Alkoxycarbonyl-, Aminocarbonyl-, Alkylaminocarbonyl-, Dialkylaminocarbonyl- oder Arylgruppe, so koennen die vorgenannten Alkyl- oder Alkoxyreste 1-7 Kohlenstoffatome, vorzugsweise 1-5 Kohlenstoffatome enthalten. Neben der Cyan-, Carboxy- und Phenylgruppe kommen vorzugsweise die Acetyl-, Propionyl-, Methoxycarbonyl-, Ethoxycarbonyl-, Aminocarbonyl-, Methylaminocarbonyl- oder Dimethylaminocarbonylgruppe in Betracht.

Bedeutet $R_4$ ein Wasserstoffatom, eine Alkyl-, Trihalogenmethyl-, Cycloalkyl-, Cycloalkenyl-, Hydroxy-, Cyan-, Carboxy-, Alkylcarbonyl-, Alkyloxycarbonyl-, Alkylaminocarbonyl- oder Dialkylaminogruppe, so enthalten die vorgenannten Alkyl- und Cycloalkylgruppen 1-7 C-Atome bzw. 3-7 C-Atome. Bevorzugte Gruppen fuer $R_4$ sind die Methyl-, Ethyl-, Isopropyl-, n-Butyl-, Trifluormethyl-, Cyclopentyl-, Cyclohexyl-, Cyclopentenyl-, Cyclohexenyl-, Hydroxy-, Cyan-, Carboxy-, Acetyl-, Propionyl-, Methyloxycarbonyl-, Ethyloxycarbonyl-, Aminocarbonyl-, Methylaminocarbonyl- und Dimethylaminocarbonylgruppe.

Bedeutet $R_4$ einen heterocyclischen Fuenfring mit 1-4 Heteroatomen oder einen heterocyclischen Sechsring mit 1-5 Heteroatomen, wobei die Heteroatome der vorgenannten Fuenf- oder Sechsringe gleich oder verschieden sein koennen und Stickstoff, Sauerstoff oder Schwefel bedeuten und gegebenenfalls an einem oder mehreren Stickstoffatomen ein Sauerstoff tragen koennen, so sind in diesem Sinne bevorzugt der Pyrrol-, Furan-, Thiophen-, Pyrazol-, Imidazol-, Thiazol-, Oxazol-, Triazol-, Tetrazol-, Thiadiazol-, Oxadiazol-, Pyrazin-, N,N-Dioxy-pyrazin-, Pyrimidin-, N,N-Dioxy-pyrimidin-, Pyridazin-, Oxazin-, Thiazin-, Triazin-, Tetrazin-, Pyridyl- und der N-Oxy-pyridylrest.

Alkyl-, Alkoxy- und Alkylmercapto-Substituenten in den heterocyclischen Fuenf- und Sechsring koennen 1-6, vorzugsweise 1-4 Kohlenstoffatome enthalten. Bevorzugt ist der Methyl-, Ethyl-, Methoxy-, Ethoxy-, Methylmercapto- und Ethylmercaptorest. Unter Halogen ist Fluor, Chlor und Brom, vorzugsweise Chlor zu verstehen.

Bedeutet $R_4$ einen Phenylring der allgemeinen Formel II so kann der Alkylteil der bei $R_5$, $R_6$ und $R_7$ genannten Substituenten 1-5 Kohlenstoffatome enthalten, vorzugsweise 1-4 Kohlenstoffatome. Bevorzugt in diesem Sinne sind beispielsweise die Methansulfonyloxy-, Ethansulfonyloxy-, n-Propansulfonyloxy-, Isopropansulfonyloxy-, Trifluormethansulfonyloxy-, Methylsulfenylmethyl-, Ethylsulfenylmethyl-, n-Propylsulfenylmethyl-, Methylsulfinylmethyl-, Ethylsulfinylmethyl-, Methylsulfonylmethyl-, Ethylsulfonylmethyl-, n-Propylsulfonylmethyl-, Methansulfonylamino-, Ethansulfonylamino-, n-Propansulfonylamino-, Trifluormethansulfonylamino-, N-Methyl-methansulfonylamino-, N-Ethyl-methansulfonylamino-, N-Methyl-ethansulfonylamino-, N-Ethyl-ethansulfonylamino-, N-Isopropyl-ethansulfonylamino-, N-Methyl-n-propansulfonylamino-, N-n-Propyl-n-propansulfonylamino-, N-Methyl-trifluormethansulfonylamino-, N-Ethyl-trifluormethansulfonylamino-, N-Isopropyl-trifluormethansulfonylamino-, Methoxycarbonyl-, Ethoxycarbonyl-, Propoxycarbonyl-, Isopropoxycarbonyl-, Methylaminocarbonyl-, Ethylaminocarbonyl-, Dimethylaminocarbonyl-, Di-n-propylaminocarbonyl-, N-Methyl-ethylaminocarbonyl-, Trifluormethyl-, Methylaminosulfonyl-, Ethylaminosulfonyl-, n-Propylaminosulfonyl-, n-Butylaminosulfonyl-, n-Pentylaminosulfonyl-, Dimethylaminosulfonyl-, Diethylaminosulfonyl-, Di-n-propylaminosulfonyl-, N-Methyl-isopropylaminosulfonyl-, Acetylamino-, Propionylamino-, Methylaminocarbonylamino-, Ethylaminocarbonylamino- oder Propylaminocarbonylaminogruppe, eine Methyl-, Ethyl-, Propyl-, Methoxy-, Ethoxy-, Propyloxy-, Allyloxy-, 2-Butenyloxy-, 3-Butenyloxy-, 2-Pentenyloxy-, Propargyloxy-, 2-Butinyloxy-, 3-Butinyloxy-, Cyanmethyloxy-, Cyanethyloxy-, Methoxycarbonylmethyloxy-, Methoxycarbonylethyloxy-, Methylmercapto-, Ethylmercapto-, Methylsulfinyl-, Ethylsulfinyl-, Methylsulfonyl- oder Ethylsulfonylgruppe.

Bei Sulfonylgruppen, die durch cyclische Iminogruppen substituiert sein koennen, sind bevorzugt die Morpholino-, Pyrrolidino-, Piperidino- und Hexamethyleniminosulfonylgruppen.

Insbesondere sind bevorzugt fuer

$R_5$ Wasserstoff, eine Alkylsulfonyloxy-, Trifluormethylsulfonyloxy-, Alkylsulfenylmethyl-, Alkylsulfinylmethyl-, Alkylsulfonylmethyl-, Alkylsulfonylamino-, N-Alkyl-alkylsulfonylamino-, Trifluormethylsulfonylamino- oder N-Alkyl-trifluormethylsulfonylaminogruppe, eine durch eine Hydroxy-, Alkoxy-, Amino-, Alkylamino oder Dialkylaminogruppe substituierte Carbonylgruppe oder eine durch eine Amino-, Dialkylamino- oder Morpholinogruppe substituierte Sulfonylgruppe, wobei jeder der vorstehend genannten Alkylteile 1 oder 2 Kohlenstoffatome enthalten kann, eine Nitro-, Cyan- oder Alkylaminosulfonylgruppe mit 1-4 Kohlenstoffatomen, eine Alkylcarbonylamino-, Aminocarbonylamino- oder N-Alkyl-aminocarbonylaminogruppe, eine Alkylmercapto-, Alkylsulfinyl- oder Alkylsulfonylgruppe, wobei jeder der vorgenannten Alkylteile 1 oder 2 Kohlenstoffatome enthalten kann, eine Halogen-, Amino-, Hydroxy-, Dialkylamino-, Alkyl-, Alkoxy-, Alkenyloxy- oder Alkinyloxygruppe vorzugsweise mit 1-3 Kohlenstoffatomen, eine Cyanmethyloxy- oder Methoxycarbonylmethyloxygruppe, die Trifluormethylgruppe oder die 1-Imidazolylgruppe,

fuer $R_6$ Wasserstoff, eine Alkylgruppe mit 1-3 Kohlenstoffatomen, eine Alkoxy- oder Dialkylaminogruppe mit 1 oder 2 Kohlenstoffatomen in jedem Alkylteil oder ein Halogenatom und

fuer $R_7$ Wasserstoff oder die Methoxygruppe.

Der Phenylteil kann 1 bis 3 der genannten Substituenten tragen.

Bevorzugte monosubstituierte Phenylverbindungen sind die Hydroxy-, $C_1$-$C_3$ Alkyl-, $C_1$-$C_3$ Alkoxy-, Allyloxy-, Propargyloxy-, Cyanmethyloxy-, Methoxycarbonylmethyloxy-, Halogen-, Nitro-, Cyan-, Aminocarbonyl-, Methoxycarbonyl-, Amino-, $C_2$-$C_6$ Dialkylamino-, $C_1$-$C_3$ Alkylmercapto-, $C_1$-$C_3$ Alkylsulfinyl-, $C_1$-$C_3$ Alkylsulfonyl-, $C_1$-$C_3$ Alkylsulfonyloxy- und die 1-Imidazolylverbindungen, wobei der Substituent in 2-, 3- oder 4-Stellung stehen kann.

Bevorzugte disubstituierte Verbindungen enthalten als Substituenten eine Alkansulfonyloxy-, Trifluormethylsulfonyloxy-, Alkylsulfenylmethyl-, Alkylsulfinylmethyl-, Alkylsulfonylmethyl-, Alkylsulfonylamino-, N-Alkyl-alkylsulfonylamino-, Trifluormethylsulfonylamino- oder N-Alkyl-trifluormethylsulfonylaminogruppe, eine durch eine Hydroxy-, Alkoxy-, Amino-, Alkylamino- oder Dialkylaminogruppe substituierte Carbonylgruppe oder eine durch eine Amino-, Dialkylamino- oder Morpholinogruppe substituierte Sulfonylgruppe, eine Alkylaminosulfonyl-, Alkylcarbonylamino-, Aminocarbonylamino- oder N-Alkylaminocarbonylaminogruppe, eine Hydroxy-, Alkyl-, Alkoxy-, Allyloxy-, Propargyloxy-, Cyanmethyloxy-, Methoxycarbonylmethyloxy-, Cyan-, Halogen-, Nitro-, Amino-, Dialkylamino-, Alkylmercapto-, Alkylsulfinyl-, Alkylsulfonyl- oder eine 1-Imidazolylgruppe, wobei die beiden Substituenten gleich oder verschieden sein koennen und in 2,3-, 2,4-, 2,5-, 2,6-, 3,4- und 3,5-Stellung bevorzugt jedoch in 2,4-, 2,5- und 3,4-Stellung stehen koennen und die vorgenannten Alkylreste, allein oder in Kombination mit anderen Resten, 1-3 C-Atome aufweisen koennen.

Bevorzugter trisubstituierter Phenylrest ist der 3,4,5-Trimethoxyphenylrest.

Fuer X ist das Sauerstoff- oder Schwefelatom bevorzugt.

Besonders bevorzugte Verbindungen sind Verbindungen der allgemeinen Formel I

in der

$R_1$ und $R_2$ gleich sind und die Methyl- oder Ethylgruppe darstellen,

$R_1$ und $R_2$ verschieden sind und Wasserstoff, die Methyl-, Ethyl-, Isopropyl-, Cyclopentyl-, Cyan-, Acetyl-, Methoxycarbonyl-, Ethoxycarbonyl-, Aminocarbonyl- und Hydrazinocarbonylgruppe bedeuten,

$R_1$ und $R_2$ einen Spirocyclopentylring darstellen, wenn $R_1$ und $R_2$ mit dem C-Atom, an das sie gebunden sind, einen Cycloalkylring bilden,

$R_3$ Wasserstoff, die Methyl-, Ethyl-, Isopropyl-, n-Butyl-, Allyl-, Cyclohexyl-, Cyclopentenyl-, Cyan-, Ethoxycarbonyl- oder Phenylgruppe bedeutet,

$R_4$ die Methyl-, Ethyl-, Isopropyl-, Trifluormethyl-, Cyclopentyl-, Hydroxy-, Cyan-, Acetyl-, Carboxy-, Ethoxycarbonyl-, Aminocarbonyl-, Methylaminocarbonyl- oder Dimethylaminocarbonylgruppe bedeutet oder

$R_4$ den Pyrrol-, Furan-, Thiophen, Pyrazol-, Imidazol-, Thiazol-, Oxazol-, Triazol-, Tetrazol-, Thiadiazol-, Oxadiazol-, Pyridin-, N-Oxy-pyridin-, Pyrazin-, N,N-Dioxy-pyrazin-, Pyrimidin-, N,N-Dioxypyrimidin-, Pyridazin-, Oxazin-, Thiazin-, Triazin- oder Tetrazinrest darstellt, sowie deren Methyl-, Ethyl-, Methoxy-, Ethoxy-, Methylmercapto-, Ethylmercapto- und chlorsubstituierten Derivate,
oder den Phenylrest der allgemeinen Formel II, in der
$R_5$ ein Wasserstoff, die Methansulfonyloxy-, Trifluormethansulfonyloxy-, Methansulfonylamino-, Trifluormethansulfonylamino-, Methansulfonyl-methylamino-, Trifluormethansulfonyl-methylamino-, Methylsulfenylmethyl-, Methylsulfinylmethyl-, Methylsulfonylmethyl-, Aminocarbonyl-, Aminosulfonyl-, Methylaminosulfonyl-, Dimethylaminosulfonyl-, Acetylamino-, Methylmercapto-, Methylsulfonyl-, Hydroxy-, Methyl-, Methoxy-, Propargyloxy-, Cyanmethyloxy-, Methoxycarbonylmethyloxy-, Cyan-, Chlor-, Nitro-, Amino-, Dimethylamino-, Trifluormethyl- oder die 1-Imidazolylgruppe,
$R_6$ Wasserstoff, die Methyl-, Methoxy-, Dimethylamino- oder Chlorgruppe bedeutet,
$R_7$ Wasserstoff oder die Methoxygruppe ist und
X fuer ein Sauerstoffatom steht.

Die Verbindungen der allgemeinen Formel I koennen nach literaturbekannten Verfahren fuer die Indol- und Oxindol-Synthese hergestellt werden.
Vergleiche hierzu:

a) P.L. Julian, E.W. Meyer und H.C. Printy, in R. C. Elderfield (Ed.), Heterocyclic Compounds, Vol. 1, S. 1-231, John Wiley and Sons, N.Y. 1952
b) R.K. Brown, in W.J. Houlihan (Ed.), Heterocyclic Compounds, Vol. 25, Part I, S. 227-537, John Wiley and Sons, N.Y. 1972.

Besonders vorteilhaft sind jedoch die in Schema 1, 2 und 3 gezeigten Synthesewege.

Schema I

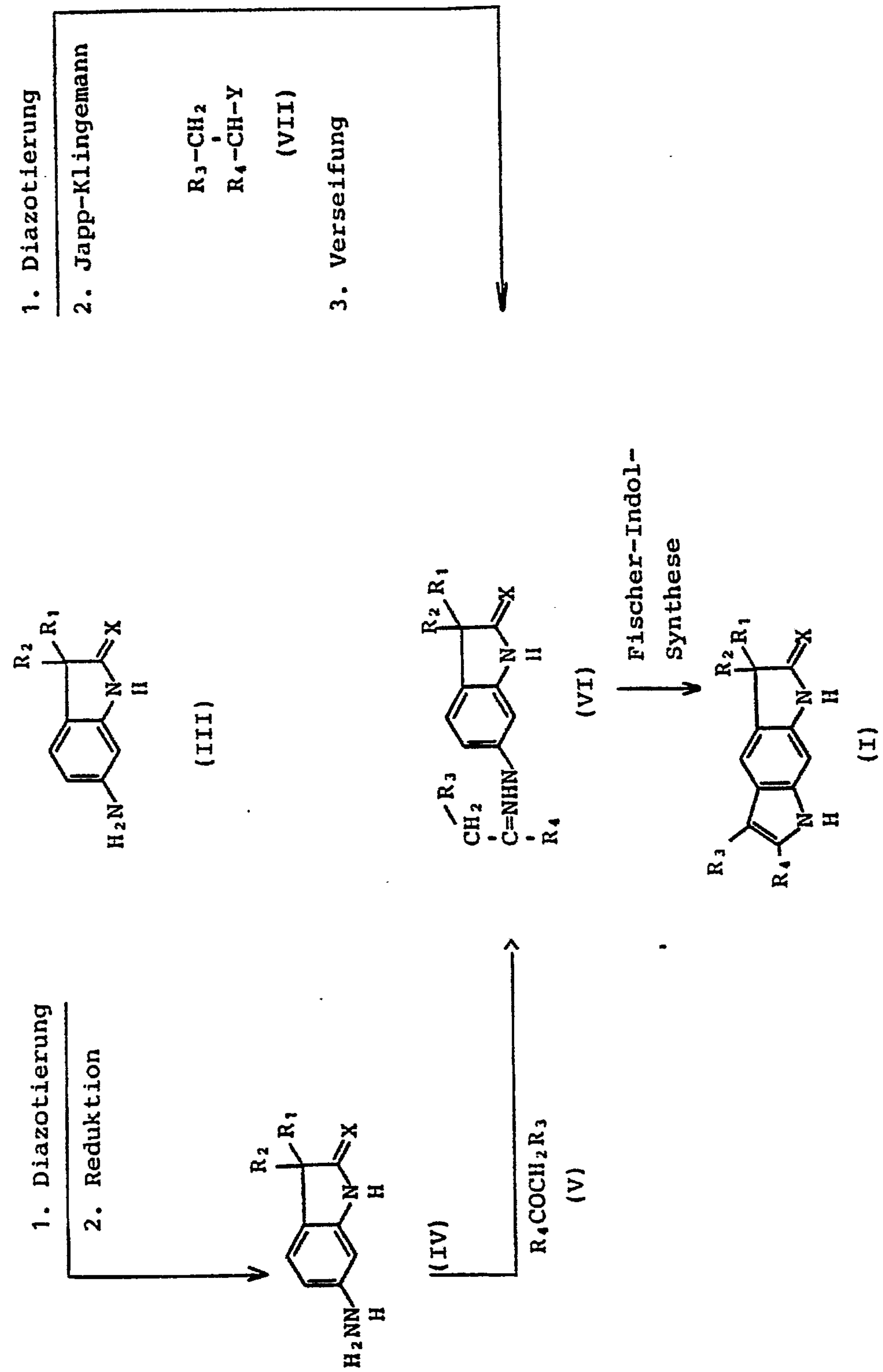


Wie aus Schema I ersichtlich lassen sich die literaturbekannten Verbindungen der Formel III (vgl. hierzu eigene Anmeldung Aktenzeichen:P34 17 643.8 und P 34 46 417.4), in der $R_1$, $R_2$ und X die angegebenen Bedeutungen haben, diazotieren, und das Diazoniumsalz zum Hydrazin IV reduzieren. Durch Umsetzung dieser Hydrazine mit Verbindungen der allgemeinen Formel V

$$R_4COCH_2R_3 \quad (V),$$

in der $R_3$ und $R_4$ die oben genannte Bedeutung haben, gelangt man zu den Hydrazonen VI, die durch eine Fischer-Indol-Synthese zu Verbindungen der allgemeinen Formel I cyclisiert werden koennen. Andererseits lassen sich die Hydrazone der allgemeinen Formel VI auch dadurch erhalten, daß man das Diazonium-Salz der Amine III in eine Japp-Klingemann-Reaktion mit Verbindungen der allgemeinen Formel VII umsetzt, wobei $R_3$ und $R_4$ die angegebene Bedeutung haben und Y ein die Methingruppe aktivierender Rest ist. Dieser Rest kann z.B. eine Aldehyd, Keton, Ester, Carbonsaeure oder Nitril sein. Die im Reaktionsgemisch intermediaer anfallende Azoverbindung wird ohne Isolierung direkt zum Hydrazon verseift.

Die Diazotierung der Amine III wird vorzugsweise unter neutralen oder sauren Bedingungen in einem polaren Loesungsmittel wie Wasser, Methanol, Ethanol, Eisessig, Salzsaeure, Schwefelsaeure oder Phosphorsaeure bei Temperaturen zwischen -70°C und 50°C vorzugsweise jedoch zwischen -5°C und 10°C durchgefuehrt. Zur Diazotierung kommen vorwiegend anorganische Salze oder organische Ester der salpetrigen Saeure in Frage wie z.B. $NaNO_2$, $KNO_2$ oder Amylnitrit.

Die Reduktion der Diazoniumsalze wird ueberwiegend in den oben genannten Loesungsmittel bei Temperaturen zwischen -50°C und den Siedepunkt des Loesungsmittels vorgenommen, vorzugsweise jedoch zwischen 0°C und 80°C, wobei als Reduktionsmittel Alkalisulfite, Schwefeldioxid, Dithionite, Zinn(II)chlorid, Zinkstaub, Eisen, Natriumamalgam, Triphenylphosphine, Endiole oder auch eine elektrochemische Reduktion in Frage kommen.

Die Umsetzung der Hydrazine mit Verbindungen der allgemeinen Formel V kann in Loesungsmitteln wie Wasser, Alkohol, Benzol, Toluol, Dioxan, DMF, Diethylether oder THF bei Temperaturen zwischen -80°C bis zum Siedepunkt des verwendeten Loesungsmittel durchgefuehrt werden. Vorteilhaft ist auch der Zusatz eine anorganische oder organische Saeure wie Salzsaeure, Schwefelsaeure, Phosphorsaeure oder Essigsaeure.

Die Japp-Klingemann-Reaktion wird vorteilhaft in den Loesungsmitteln durchgefuehrt, in denen die bereits oben beschriebene Diazotierung durchgefuehrt werden kann. Dies sind also insbesondere Wasser, Methanol, Ethanol, Eisessig, Salzsaeure, Schwefelsaeure oder Phosphorsaeure bei Temperaturen zwischen -50°C bis 80°C vorzugsweise jedoch zwischen 0°C und 25°C. Die nachfolgende Verseifung kann thermisch oder nach Zusatz eine Base oder Saeure wie z.B. Natronlauge, Kalilauge, Salzsaeure, Schwefelsaeure, Phosphorsaeure oder Eisessig bei Temperaturen bis zum Siedepunkt des Loesungsmittels durchgefuehrt werden.

Die Fischer-Indol-Synthese der Hydrazone VI wird ohne Loesungsmittel oder in einem Solvens wie Alkohol, Nitrobenzol, Essigsaeure, Xylol, Cumol, Toluol thermisch oder in Gegenwart eines sauren Katalysator, der jedoch auch Loesungsmittel sein kann, durchgefuehrt,wobei Salzsaeure, Schwefelsaeure, Phosphorsaeure, Polyphosphorsaeure, Eisessig, Ameisensaeure, Zink(II)chlorid, Bortrifluorid, Kationenaustauscher, Sulfosalicylsaeure oder Polyphosphatester in Frage kommen bei Temperaturen zwischen 0°C und dem Siedepunkt des verwendeten Loesungsmittels.

Die Hydrazone VI lassen sich auch aus den Aminen III nach Schema 2 herstellen.

Schema 2

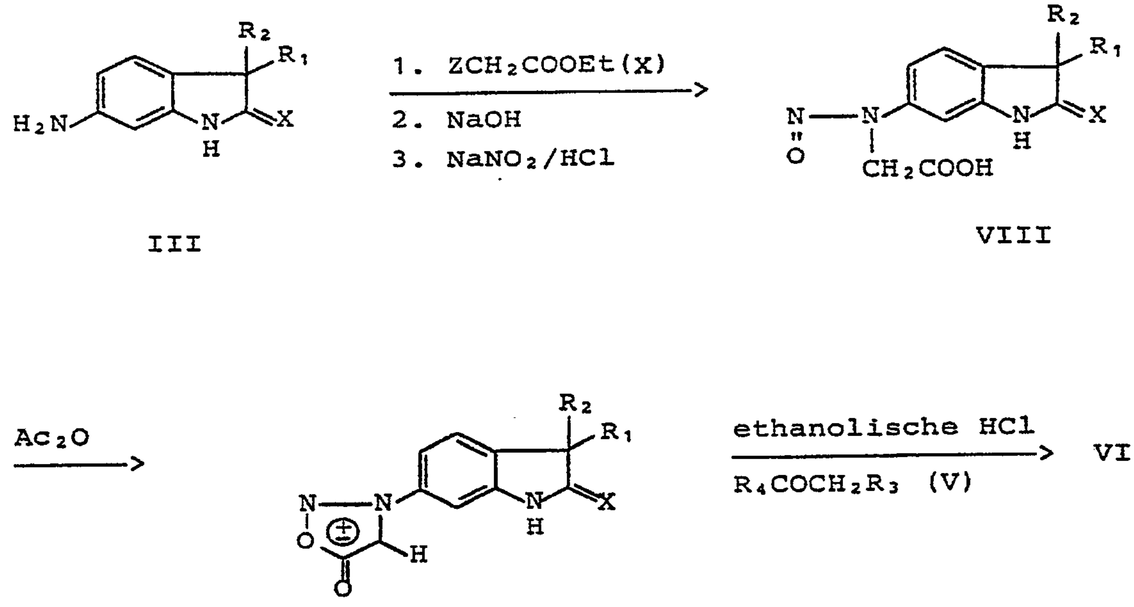


III

VIII

IX

VI

Die Umsetzung von Verbindung III mit Halogenessigestern X, in den als Halogen F, Cl, Br oder J, vorzugsweise jedoch Br, in Frage kommen, wird vorteilhaft in polaren oder unpolaren Loesungsmittel wie z.B. Methylenchlorid, Toluol, Dioxan, Alkoholen oder Dimethylformamid bei Temperaturen zwischen -50°C und dem Siedepunkt des Loesungsmittels, vorzugsweise zwischen 25°C und 100°C, durchgefuehrt.

Die so erhaltenen Ester koennen nach wohlbekannten Verfahren z.B. mit anorganischen Basen wie z.B. Natriumhydroxid, Kaliumhydroxid, Natriumhydrogencarbonat oder Kaliumhydrogencarbonat in protischen Loesungsmitteln wie Wasser oder Alkohol oder mit anorganischen oder organischen Saeuren wie Salzsaeure, Schwefelsaeure, Eisessig, Phosphorsaeure oder Polyphosphorsaeure gegebenenfalls unter Zugabe eines Loesungsmittels wie Wasser oder Alkohol verseift werden.

Die Nitrosierung der erhaltenen Saeuren zu den Verbindungen der allgemeinen Formel VIII wird vorzugsweise unter neutralen oder sauren Bedingungen in einem polaren Loesungsmittel wie Wasser, Methanol, Ethanol, Eisessig, Salzsaeure, Schwefelsaeure oder Phosphorsaeure bei Temperaturen zwischen -70°C und 50°C vorzugsweise jedoch zwischen -5°C und 10°C durchgefuehrt. Zur Nitrosierung kommen vorwiegend anorganische Salze oder organische Ester der salpetrigen Saeure in Frage wie z.B. $NaNO_2$, $KNO_2$ oder Amylnitrit.

Die Umsetzung der N-Nitroso-carbonsaeuren VIII zu den Sydnonen IX gelingt in inerten Loesungsmittel wie z.B. Dioxan, Diethylether, Tetrahydrofuran, Toluol mit wasserentziehenden Reagenzien wie z.B. Acetanhydrid, Propionsaeureanhydrid, Schwefelsaeure, Phosphorpentoxid, $PCl_5$ oder $PCl_3$ bei Temperaturen zwischen -50°C und dem Siedepunkt des Loesungsmittels, vorteilhaft jedoch zwischen 25°C und 100°C.

Die Sydnone IX lassen sich unter sauren Bedingungen in die Hydrazine IV zerlegen, die in situ mit den Ketonen V zu den Hydrazonen VI abgefangen werden. Als Saeuren fuer die Verseifung der Sydnone kommen Salzsaeure, Schwefelsaeure, Phosphorsaeure, Polyphosphorsaeure oder organische Saeuren wie Eisessig bei Temperaturen zwischen -70°C und 100°C vorzugsweise zwischen 0°C und 70°C in Betracht.

Alternativ zu Schema 1 und 2 lassen sich die Verbindungen der allgemeinen Formel I auch nach dem in Schema 3 vorgestellten Weg der Oxindol-Synthese herstellen (vgl. P.L. Julian, E.W. Meyer und H.C. Printy, in R.C. Elderfield (Ed.), Heterocyclic Compounds, Vol. 3, John Wiley + Sons, New York 1952, S. 128-142).

Hinsberg-Synthese: Umsetzung aromatischer Amine mit den Bisulfit-Additions-Verbindungen von Ketonen.

Brunner-Synthese: Cyclisierung aromatischer Amine ueber das Hydrazid zu Oxindolen.

Stollé-Synthese: Cyclisierung aromatischer Amine ueber ein Amid zum Oxindol.

Schema 3

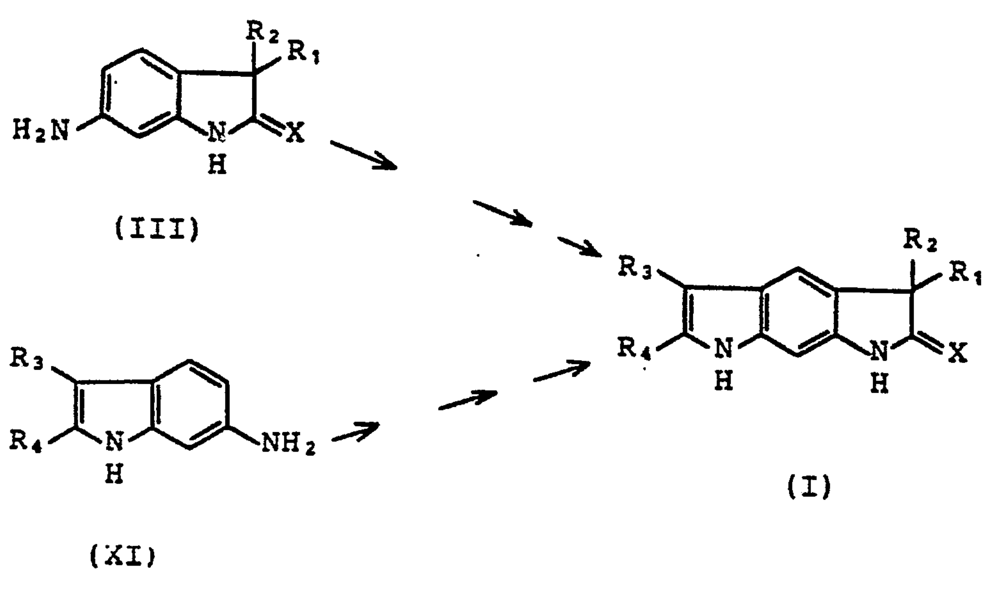

(III)

(XI)

(I)

Die Oxindolsynthese von Verbindungen der allgemeinen Formel III zu Verbindungen der allgemeinen Formel I gilt nur fuer den Fall, wo $R_4$ die Hydroxygruppe bedeutet. Die Verbindungen der allgemeinen Formel (XI) sind zum Teil neu und koennen nach literaturbekannten Verfahren hergestellt werden.

Verbindungen der allgemeinen Formel I koennen auch nachtraeglich in eine andere Verbindung der Formel I umgewandelt werden. Dies trifft zum Beispiel zu

a) fuer die Oxidation eines Fuenf- oder Sechsringes mit einem oder mehreren Stickstoffatomen zu den entsprechenden N-Oxiden. Die Oxidation wird zweckmaeßig mit einem oder mehreren Aequivalenten des verwendeten Oxidationsmittels durchgefuehrt, z.B. mit Wasserstoffperoxid in Eisessig, Trifluoressigsaeure oder in Ameisensaeure bei 20-100°C oder in Aceton bei 0-60°C, mit einer Persaeure wie Perameisensaeure oder m-Chlorperbenzoesaeure in Eisessig, Trifluoressigsaeure, Methylenchlorid oder Chloroform bei Temperaturen zwischen 0 und 60°C.

b) fuer die Herstellung von Verbindungen der allgemeinen Formel I, in der $R_4$ einen Rest der allgemeinen Formel II bedeutet und $R_5$ eine Alkylsulfinyl-, Alkylsulfonyl-, Alkylsulfinylmethyl- oder Alkylsulfonylmethylgruppe darstellt, durch nachtraegliche Oxidation einer Verbindung der allgemeinen Formel XII

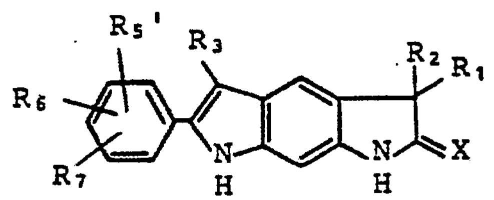

(XII),

in der

$R_1$, $R_2$, $R_3$, $R_6$, $R_7$ und X wie eingangs definiert sind und $R_5'$ eine Alkylmercapto- oder Alkylsulfenylmethylgruppe mit jeweils 1-3 Kohlenstoffatomen im Alkylteil ist.

Die Oxidation wird vorzugsweise in einem Loesungsmittel oder Loesungsmittelgemisch, z.B. in Wasser, Wasser/Pyridin, Aceton, Eisessig, verduennter Schwefelsaeure oder Trifluoressigsaeure, je nach dem verwendeten Oxidationsmittel zweckmaeßigerweise bei Temperaturen zwischen -80 und 100°C durchgefuehrt.

Zur Herstellung einer Alkylsulfinyl- oder Alkylsulfinylmethylverbindung der allgemeinen Formel I wird die Oxidation zweckmaeßigerweise mit einem Aequivalent des verwendeten Oxidationsmittel durchgefuehrt, z.B. mit Wasserstoffperoxid in Eisessig, Trifluoressigsaeure oder Ameisensaeure bei 0 bis 20°C oder in Aceton bei 0 bis 60°C, mit einer Persaeure wie Perameisensaeure in Eisessig oder Trifluoressigsaeure bei 0 bis 50°C oder mit m-Chlorperbenzoesaeure in Methylenchlorid oder Chloroform bei -20 bis 60°C, mit Natriummetaperjodat in waessrigem Methanol oder Ethanol bei -15 bis 25°C, mit Brom in Eisessig oder waessriger Essigsaeure, mit N-Brom-succinimid in Ethanol, mit tert.-Butyl-hypochlorid in Methanol bei -80 bis -30°C, mit Jodbenzodichlorid in waessrigem Pyridin bei 0 bis 50°C, mit Salpetersaeure in Eisessig bei 0 bis 20°C, mit Chromsaeure in Eisessig oder in Aceton bei 0 bis 20°C und mit Sulfurylchlorid in Methylenchlorid bei -70°C, der hierbei erhaltene Thioether-Chlor-Komplex wird zweckmaeßigerweise mit waessrigem Ethanol hydrolysiert.

Zur Herstellung einer Alkylsulfonyl- oder Alylsulfonylmethylverbindung der allgemeinen Formel I wird die Oxidation zweckmaeßigerweise mit einem bzw. mit zwei oder mehr Aequivalenten des verwendeten Oxidationsmittels durchgefuehrt, z.B. Wasserstoffperoxid in Eisessig, Trifluoressigsaeure oder in Ameisensaeure bei 20 bis 100°C oder in Aceton bei 0 bis 60°C, mit einer Persaeure wie Perameisensaeure oder m-Chlorperbenzoesaeure in Eisessig, Trifluoressigsaeure, Methylenchlorid oder Chloroform bei Temperaturen zwischen 0 und 60°C, mit Salpetersaeure in Eisessig bei 0 bis 20°C, mit Chromsaeure oder Kaliumpermanganat in Eisessig, Wasser/Schwefelsaeure oder in Aceton bei 0 bis 20°C.

c) fuer die Herstellung von Verbindungen der allgemeinen Formel I, in der $R_4$ einen Rest der allgemeinen Formel II bedeutet und $R_5$ eine Alkansulfonyloxy-, Trifluormethansulfonyloxy-, Alkansulfonylamino-, N-Alkyl-alkansulfonylamino-, Trifluormethansulfonylamino-, oder N-Alkyl-trifluormethansulfonylaminogruppe darstellt, durch die nachtraegliche Umsetzung einer Verbindung der allgemeinen Formeln XIII

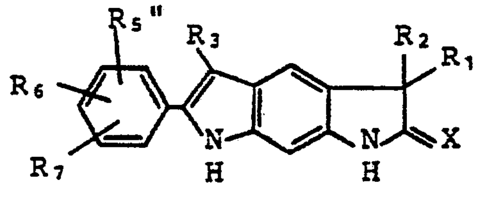

(XIII),

in der

$R_1$, $R_2$, $R_3$, $R_6$, $R_7$ und X wie eingangs definiert sind und $R_5''$ eine Hydroxy-, Amino- oder N-Alkylaminogruppe mit 1-3 Kohlenstoffatomen im Alkylteil darstellt, mit einer Sulfonsaeure der allgemeinen Formel XIV

$R_8$-$SO_2OH$ (XIV)

in der
$R_8$ eine Alkylgruppe mit 1-3 Kohlenstoffatomen oder eine Trifluormethylgruppe darstellt, in Gegenwart eines wasserentziehenden und/oder die Saeure oder das Amin aktivierenden Mittels oder mit deren reaktionsfaehigen Derivaten.
Die Umsetzung wird zweckmaeßigerweise in einem Loesungsmittel oder Loesungsmittelgemisch wie Methylenchlorid, Ether, Tetrahydrofuran, Dioxan oder Benzol gegebenenfalls in Gegenwart eines saeurebindenden Mittels wie Natriumcarbonat, Triethylamin oder Pyridin, wobei die beiden letzteren gleichzeitig auch als Loesungsmittel verwendet werden koennen, in Gegenwart eines die Saeure aktivierenden oder wasserentziehenden Mittels wie Thionylchlorid oder Phosphorpentachlorid, vorzugsweise jedoch mit einem reaktionsfaehigen Derivat einer Verbindung der allgemeinen Formel XIV, z.B. mit deren Anhydrid oder Halogenid wie Methansulfonsaeurechlorid oder Ethansulfonsaeurechlorid, vorzugsweise bei Temperaturen zwischen 0 und 100°C, z.B. bei Temperaturen zwischen Raumtemperatur und 50°C, durchgefuehrt.

d) fuer die Herstellung von Verbindungen der allgemeinen Formel I, in der $R_4$ einen Rest der allgemeinen Formel II bedeutet und $R_2$ und/oder $R_5$ eine durch eine Amino-, Alkylamino-, Dialkylamino- oder Hydrazinogruppe substituierte Carbonyl- oder Sulfonylgruppe darstellt, durch die nachtraegliche Umsetzung einer Verbindung der allgemeinen Formel XV

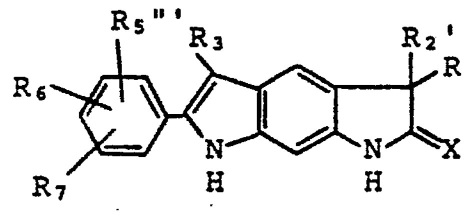

(XV) ,

in der
$R_1$, $R_3$, $R_6$, $R_7$ und X wie eingangs definiert sind und $R_2$' und/oder $R_5$''' eine Carboxyl- oder Hydroxysulfonylgruppe darstellen, oder ein reaktionsfaehiges Derivat hiervon wie z.B. Ester oder Saeurechlorid mit Hydrazin oder einem Amin der allgemeinen Formel XVI

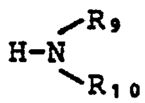

(XVI) ,

in der
$R_9$ und $R_{10}$, die gleich oder verschieden sein koennen, Wasserstoffatome oder Alkylgruppen mit 1-5 Kohlenstoffatomen darstellen, oder mit einem reaktionsfaehigen Derivat hiervon, falls $R_2$' und/oder $R_5$''' die Carboxyl- oder Hydroxysulfonylgruppe darstellen.
Die Umsetzung wird zweckmaeßigerweise in einem Loesungsmittel oder Loesungsmittelgemisch wie Methylenchlorid, Ethanol, Chloroform, Tetrachlorkohlenstoff, Ether, Tetrahydrofuran, Dioxan, Benzol, Toluol, Acetonitril oder Dimethylformamid, gegebenenfalls in Gegenwart eines die Saeure aktivierenden Mittels oder eines wasserentziehenden Mittels, z.B. in Gegenwart von Chlorameisensaeureethylester, Thionylchlorid, Phosphortrichlorid, Phosphorpentoxid, N'N-Dicyclohexylcarbodiimid/N-Hydroxysuccinimid, N,N'-Carbonyldiimidazol oder N,N'-Thionyldiimidazol oder Triphenylphosphin/Tetrachlorkohlenstoff, oder eines die Hydrazino- oder Aminogruppe aktivierender Mittels, z.B. Phosphortrichlorid, und gegebenenfalls in Gegenwart einer anorganischen Base wie Natriumcarbonat oder einer tertiaeren organischen Base wie Triethylamin oder Pyridin, welche gleichzeitig als Loesungsmittel dienen koennen, bei Temperaturen zwischen -25 und 250°C, vorzugsweise jedoch bei Temperaturen zwischen -10°C und der Siedetemperatur des verwendeten Loesungsmittels durchgefuehrt werden, desweiteren kann waehrend der Umsetzung entstehendes Wasser durch azeotrope Destillation, z.B. durch Erhitzen mit Toluol am Wasserabscheider, oder durch Zugabe eines Trockenmittels wie Magnesiumsulfat oder Molekularsieb abgetrennt werden.
Besonders vorteilhaft wird jedoch die Umsetzung in einem entsprechenden Halogenid, z.B. dem Carbonsaeure- oder Sulfonsaeurechlorid, und Hydrazin oder einem entsprechenden Amin, wobei diese gleichzeitig als Loesungsmittel dienen koennen, und bei Temperaturen zwischen 0 und 50°C durchgefuehrt.

e) fuer die Herstellung von Verbindungen der allgemeinen Formel I, in der $R_3$ eine Cyangruppe bedeutet, durch nachtraegliche Umsetzung einer Verbindung der allgemeinen Formel I, in der $R_1$, $R_2$, $R_4$ und X die angegebene Bedeutung haben, und $R_3$ Wasserstoff bedeutet, mit N-Carbonylsulfamoylchlorid, das auch als Chlorsulfonylisocyanat bezeichnet wird, in einem geeigneten Loesungsmittel nach an sich bekannten Verfahren (Chem.Ber. 100, 2719 (1967); Synthesis 1978, 374 und J.Chem.Soc., Perkin I, 1978, 1117).
Die Reaktion wird zweckmaeßig in einem unter den Reaktionsbedingungen inerten Loesungsmittel, z.B. Wasser, Methanol, Ethanol, n-Butanol, Dioxan, Acetonitril, Nitromethan, Pyridin, Dimethylformamid

oder Methylenchlorid, gegebenenfalls, in Gegenwart eines saeurebindenden Mittels durchgefuehrt.
Die Reaktionen werden unter Eiskuehlung, bei Raumtemperatur oder unter Erwaermen, gegebenenfalls unter einer Schutzgasatmosphaere, ausgefuehrt.

f) fuer die Herstellung von Verbindungen der allgemeinen Formel I, in der $R_3$ und $R_4$ die Carboxy-, Alkoxycarbonyl- oder Aminocarbonylgruppe bedeutet, oder $R_4$ ein Rest der allgemeinen Formel II ist, wobei $R_5$ eine Carboxy-, Alkoxycarbonyl-, Aminocarbonyl-, Alkoxycarbonylalkyloxy- oder Carboxyalkyloxygruppe bedeutet, durch nachtraegliche Alkoholyse und/oder Hydrolyse von Verbindungen der allgemeinen Formel I, in der $R_3$ und $R_4$ die Cyangruppe bedeutet oder $R_4$ ein Rest der allgemeinen Formel II ist, wobei $R_5$ eine Cyan- oder Cyanalkyloxygruppe darstellt.
Die nachtraegliche Alkoholyse und/oder Hydrolyse wird zweckmaeßigerweise entweder in Gegenwart einer Saeure wie Salzsaeure, Schwefelsaeure, Phosphorsaeure oder Trichloressigsaeure oder in Gegenwart einer Base wie Natriumhydroxid oder Kaliumhydroxid in einem geeigneten Loesungsmittel wie Wasser, Wasser/Methanol, Ethanol, Wasser/Ethanol, Wasser/Isopropanol oder Wasser/Dioxan bei Temperaturen zwischen -10 und 120°C, z.B., bei Temperaturen zwischen Raumtemperatur und der Siedetemperatur des Reaktionsgemisches, durchgefuehrt.

g) fuer die Herstellung von Verbindungen der allgemeinen Formel I, in der X ein Schwefelatom bedeutet, durch nachtraegliche Einfuehrung des Schwefelatoms in Verbindungen der allgemeinen Formel I, in der $R_1$, $R_2$, $R_3$ und $R_4$ die angegebene Bedeutung haben und X ein Sauerstoffatom ist.

Die Umsetzung wird nach literaturbekannten Verfahren mit einem das Schwefelatom uebertragenden Reagenz, wie z.B. Phosphorpentasulfid durchgefuehrt, wobei man zweckmaeßigerweise 1-5 Mol $P_2S_5$ vorzugsweise jedoch 1 Mol in einem geeigneten Loesungsmittel umsetzt. Als Loesungsmittel kommen Tetrahydrofuran, Dioxan, Benzol, Toluol oder Pyridin bei Temperaturen zwischen 25 und 125°C in Betracht.

Bevorzugt ist jedoch Pyridin bei einer Reaktionsdauer von 1-10 Stunden, vorzugsweise jedoch 5 Stunden in Abhaengigkeit vom Reaktionspartner.

Ferner koennen die erhaltenen Verbindungen der allgemeinen Formel I anschließend gewuenschtenfalls in ihre physiologisch vertraeglichen Saeureadditionssalze mit anorganischen oder organischen Saeuren ueberfuehrt werden. Als Saeuren kommen hierfuer beispielsweise Salzsaeure, Bromwasserstoffsaeure, Schwefelsaeure, Phosphorsaeure, Fumarsaeure, Bernsteinsaeure, Weinsaeure, Zitronensaeure, Milchsaeure, Maleinsaeure oder Methansulfonsaeure in Betracht.

Wie bereits eingangs erwaehnt weisen die neuen Verbindungen der allgemeinen Formel I, deren Tautomere und deren physiologisch vertraegliche Saeureadditionssalze bei einer langen Wirkungsdauer ueberlegene pharmakologische Eigenschaften auf, insbesondere eine blutdrucksenkende und/oder positivinotrope Wirkung und/oder beeinflussen sie die Thrombozytenfunktion und verbessern die Mikrozirkulation.

Zur Herstellung von Arzneimitteln werden die Substanzen der allgemeinen Formel I in an sich bekannter Weise mit geeigneten pharmazeutischen Traegersubstanzen, Aroma-, Geschmacks- und Farbstoffen gemischt und beispielsweise als Tabletten oder Dragees ausgeformt oder unter Zugabe entsprechender Hilfsstoffe in Wasser oder Oel, wie z.B. Olivenoel, suspendiert oder geloest.
Die erfindungsgemaeßen neuen Substanzen der allgemeinen Formel I und ihre Salze koennen in fluessiger oder fester Form enteral oder parenteral appliziert werden. Als Injektionsmedium kommt vorzugsweise Wasser zur Anwendung, welches die bei Injektionsloesungen ueblichen Zusaetze wie Stabilisierungsmittel, Loesungsvermittler oder Puffer enthaelt.

Derartige Zusaetze sind z.B. Tartrat- und Citratpuffer, Ethanol, Komplexbildner (wie Ethylendiamintetraessigsaeure und deren nicht toxische Salze) und hochmolekulare Polymere (wie fluessiges Polyethylenoxid) zur Viskositeatsregulierung. Feste Traegerstoffe sind z.B. Staerke, Lactose, Mannit, Methylcellulose, Talkum, hochdisperse Kieselsaeuren, hochmolekulare Fettsaeuren (wie Stearinsaeure), Gelatine, Agar-Agar, Calciumphosphat, Magnesiumstearat, tierische und pflanzliche Fette und feste hochmolekulare Polymere (wie Polyethylenglykole). Fuer orale Applikation geeignete Zubereitungen koennen gewuenschtenfalls Geschmacks- und Sueßstoffe enthalten.

Die erfindungsgemaeßen Verbindungen werden ueblicherweise in Mengen von 10-500 mg pro Tag bezogen auf 75 kg Koerpergewicht appliziert. Bevorzugt ist es, 2-3 mal pro Tag 1-2 Tabletten mit einem Wirkstoffgehalt von 5-200 mg zu verabreichen. Die Tabletten koennen auch retardiert sein, wodurch nur noch 1 mal pro Tag 1-2 Tabletten mit 10-500 mg Wirkstoff gegeben werden muß. Der Wirkstoff kann auch durch Injektion 1-8 mal pro Tag bzw. durch Dauerinfusion gegeben werden, wobei Mengen von 5-200 mg/Tag normalerweise ausreichen.

Bevorzugt im Sinne der vorliegenden Erfindung sind außer den in den Beispielen genannten Verbindungen die folgenden und deren Tautomere.
3,3-Dimethyl-6-(pyrrol-2-yl)-benzo[1,2-b:5,4-b']dipyrrol-2-(1H,3H,7H)-on
3,3-Dimethyl-6-(thiazol-4-yl)-benzo[1,2-b:5,4-b']dipyrrol-2(1H,3H,7H)-on
3,3-Dimethyl-6-(1,2,4-triazol-3-yl)-benzo[1,2-b:5,4-b']dipyrrol-2(1H,3H,7H)-on
3,3-Dimethyl-6-(5-carboxy-1,2,3-triazol-4-yl)-benzo[1,2-b:5,4-b']dipyrrol-2(1H,3H,7H)-on
3,3-Dimethyl-6-(5-methylmercapto-1,3,4-oxadiazol-2-yl)-benzo[1,2-b:5,4-b']dipyrrol-2(1H,3H,7H)-on
3,3-Dimethyl-6-(1,2,5-thiadiazol-3-yl)-benzo[1,2-b:5,4-b']dipyrrol-2(1H,3H,7H)-on
3,3-Dimethyl-6-(pyrimidin-5-yl)-benzo[1,2-b:5,4-b']dipyrrol-2(1H,3H,7H)-on

3,3-Dimethyl-6-(pyrazin-2-yl)-benzo[1,2-b:5,4-b']dipyrrol-2(1H,3H,7H)-on
3,3-Dimethyl-6-(N-oxy-4-pyridyl)-benzo[1,2-b:5,4-b']dipyrrol-2(1H,3H,7H)-on
3,3-Dimethyl-6-(4-nitro-phenyl)-benzo[1,2-b:5,4-b']dipyrrol-2(1H,3H,7H)-on
3,3-Dimethyl-6-(4-methylmercapto-phenyl)-benzo[1,2-b:5,4-b']dipyrrol-2-(1H,3H,7H)-on
3,3-Dimethyl-6-(4-amino-phenyl)-benzo[1,2-b:5,4-b']dipyrrol-2(1H,3H,7H)-on
3,3-Dimethyl-6-(4-methylsulfenyl-2-methoxy-phenyl)-benzo[1,2-b:5,4-b']dipyrrol-2(1H,3H,7H)-on
3,3-Dimethyl-6-(4-methylsulfinyl-2-methoxy-phenyl)-benzo[1,2-b:5,4-b']dipyrrol-2(1H,3H,7H)-on
3,3-Dimethyl-6-(4-methylsulfonyl-2-methoxy-phenyl)-benzo[1,2-b:5,4-b']dipyrrol-2(1H,3H,7H)-on
3,3-Dimethyl-6-(4-methylsulfonyloxy-2-methoxy-phenyl)-benzo[1,2-b:5,4-b']dipyrrol-2(1H,3H,7H)-on
3,3,5,6-Tetramethyl-benzo[1,2-b:5,4-b']dipyrrol-2(1H,3H,7H)-on
3,3,5-Trimethyl-benzo[1,2-b:5,4-b']dipyrrol-2(1H,3H,7H)-on
3,3,6-Trimethyl-5-cyan-benzo[1,2-b:5,4-b']dipyrrol-2(1H,3H,7H)-on
3,3,6-Trimethyl-5-aminocarbonyl-benzo[1,2-b:5,4-b']dipyrrol-2(1H,3H,7H)-on
3,3,6-Trimethyl-5-ethoxycarbonyl-benzo[1,2-b:5,4-b']dipyrrol-2(1H,3H,7H)-on
3,3-Dimethyl-6-trifluormethyl-benzo[1,2-b:5,4-b']dipyrrol-2(1H,3H,7H)-on
3,3-Dimethyl-6-(cyclohexen-1-yl)-benzo[1,2-b:5,4-b']dipyrrol-2(1H,3H,7H)-on
3,3-Dimethyl-6-cyclopropyl-benzo[1,2-b:5,4-b']dipyrrol-2(1H,3H,7H)-on
3,3-Dimethyl-6-cyan-benzo[1,2-b:5,4-b']dipyrrol-2(1H,3H,7H)-on
3,3-Dimethyl-6-acetyl-benzo[1,2-b:5,4-b']dipyrrol-2(1H,3H,7H)-on
3,3-Dimethyl-5,6-diphenyl-benzo[1,2-b:5,4-b']-dipyrrol-2(1H,3H,7H)-on
3,3-Dimethyl-6-(3,4,5-trimethoxy-phenyl)-benzo[1,2-b:5,4-b']dipyrrol-2(1H,3H,7H)-on
3,3-Diethyl-6-(4-dimethylamino-phenyl)-benzo[1,2-b:5,4-b']dipyrrol-2(1H,3H,7H)-on
3,3-Diethyl-6-(2-hydroxy-5-pyrimidinyl)-benzo[1,2-b:5,4-b']dipyrrol-2(1H,3H,7H)-on
3,3-Diethyl-6-(4-pyrimidinyl)-benzo[1,2-b:5,4-b']dipyrrol-2(1H,3H,7H)-on
3,3-Diethyl-6-(3-hydroxy-6-pyridazinyl)-benzo[1,2-b:5,4-b']dipyrrol-2(1H,3H,7H)-on
6'-(2-Methyl-4-oxazolyl)-spiro[cyclopentan-1,3'-benzo[1',2'-b:5',4'-b']dipyrrol]-2'(1'H,3'H,7'H)-on
6'-(2-Chlor-4-pyridyl)-spiro[cyclopentan-1,3'-benzo[1',2'-b:5',4'-b']dipyrrol]-2'(1'H,3'H,7'H)-on
6'-(2-Methyl-4-pyridyl)-spiro[cyclopentan-1,3'-benzo[1',2'-b:5',4'-b']dipyrrol]-2'(1'H,3'H,7'H)-on
3-Isopropyliden-6-(2-pyridyl)-benzo[1,2-b:5,4-b']dipyrrol-2(1H,3H,7H)-on
3-Methyl-3-ethoxycarbonyl-5-methyl-6-phenyl-benzo[1,2-b:5,4-b']dipyrrol-2(1H,3H,7H)-on
3-Methyl-3-ethoxycarbonyl-6-(4-trifluormethylsulfonyl-2-methoxy-phenyl)-benzo[1,2-b:5,4-b']dipyrrol-2(1H,3H,7H)-on
3-Methyl-3-ethoxycarbonyl-6-(4-morpholinylsulfonyl-2-methoxy-phenyl)-benzo[1,2-b:5,4-b']dipyrrol-2(1H,3H,7H)-on
3-Methyl-6-(2-furanyl)-benzo[1,2-b:5,4-b']dipyrrol-2(1H,3H,7H)-on
3-Methyl-6-(imidazol-4-yl)-benzo[1,2-b:5,4-b']dipyrrol-2(1H,3H,7H)-on
3-Methyl-6-(2,4-dimethoxy-phenyl)-benzo[1,2-b:5,4-b']dipyrrol-2(1H,3H,7H)-on
3-Methyl-6-(2-methoxy-4-aminosulfonyl-phenyl)-benzo[1,2-b:5,4-b']dipyrrol-2(1H,3H,7H)-on
3-Methyl-6-(2-methoxy-4-methylsulfonylamino-phenyl)-benzo[1,2-b:5,4-b']dipyrrol-2(1H,3H,7H)-on
3-Methyl-6-(2-methoxy-4-methylsulfenylmethyl-phenyl)-benzo[1,2-b:5,4-b']dipyrrol-2(1H,3H,7H)-on
3-Methyl-6-(2-methoxy-4-methylsulfinylmethyl-phenyl)-benzo[1,2-b:5,4-b']dipyrrol-2(1H,3H,7H)-on
3-Methyl-6-(2-methoxy-4-methylsulfonylmethyl-phenyl)-benzo[1,2-b:5,4-b']dipyrrol-2(1H,3H,7H)-on
6-(2-methoxy-4-cyanmethoxy-phenyl)-benzo[1,2-b:5,4-b']clipyrrol-2(1H,3H,7H)-on
3-Ethyl-6-(2-methoxy-4-propargyloxy-phenyl)-benzo[1,2-b:5,4-b']dipyrrol-2(1H,3H,7H)-on
3-Ethyl-6-(4-pyridyl)-benzo[1,2-b:5,4-b']dipyrrol-2(1H,3H,7H)-on
7-Cyclopentyl-6-(4-pyridyl)-benzo[1,2-b:5,4-b']dipyrrol-2(1H,3H,7H)-on
3,3,5-Trimethyl-benzo[1,2-b:5,4-b']dipyrrol-2,6(1H,3H,5H,7H)-dion

Beispiel 1

3,3-Dimethyl-6-(4-pyridyl)-benzo[1,2-b:5,4-b']dipyrrol-2(1H,3H,7H)-on

a) 6.8 g (38 mmol) 6-Amino-3,3-dimethyl-indolin-2-on werden in 125 ml 50proz. Schwefelsaeure geloest und unterhalb von 5°C 2.8 g (40.6 mmol) Natriumnitrit, geloest in 25 ml Wasser, zugetropft. Nach 15 Minuten werden 0.5 g Harnstoff zugesetzt und nochmals 15 Minuten nachgeruehrt. In die Loesung werden bei 5°C 26.35 g (116.8 mmol) Zinn(II)chlorid Dihydrat, geloest in 20 ml konzentrierter Salzsaeure, zugetropft. Nach zwei Stunden wurde die Loesung mit 6.9 g (57 mmol) 4-Acetylpyridin versetzt, zwei Stunden bei 25°C geruehrt, der Rueckstand abgesaugt, in Wasser suspendiert, mit waessrigem Ammoniak neutralisiert und abgesaugt. Man erhaelt 8.5 g (76 %) 4-Acetylpyridin-(3,3-dimethyl-2-oxo-indolin-6-hydrazon) vom Schmp. 272-74°C.

b) 8.3 g (28.2 mmol) des so erhaltenen Hydrazons werden mit 50 ml Polyphosphorsaeure unter Stickstoffatmosphaere 6 Stunden bei 110-120°C Oelbadtemperatur geruehrt. Der gekuehlte Ansatz wird mit Eiswasser durchgearbeitet und die Suspension mit konzentriertem Ammoniak neutralisiert und abgesaugt. Der Rueckstand wurde aus Methanol/Methylenchlorid umkristallisiert. Man erhaelt 5.18 g (66 %) der Titelverbindung vom Schmp. >300°C.

Analog zu Beispiel 1 erhaelt man:

| Bezeichnung | Ausbeute [%] | Schmp. [°C] Lsg.-mittel |
|---|---|---|
| 3,3-Dimethyl-6-(3-pyridyl)-benzo[1,2-b:5,4-b']-dipyrrol-2(1H,3H,7H)-on x 1 mol $CH_3OH$ aus 3-Acetylpyridin-(3,3-dimethyl-2-oxoindolin-6-hydrazon) als Rohprodukt | 24 | 305 Methanol |
| 3,3-Dimethyl-6-(pyridazin-4-yl)-benzo[1,2-b:5,4-b']dipyrrol-2(1H,3H,7H)-on x 1 mol $CH_3OH$ aus 4-Acetyl-pyridazin-(3,3-dimethyl-2-oxo-indolin-6-hydrazon) Schmp. 240°C | 33 | 330 Methanol |
| 3,3-Dimethyl-6-[4-(1H-imidazol-1-yl)-phenyl]-benzo[1,2-b:5,4-b']dipyrrol-2-(1H,3H,7H)-on aus 4-(1H-imidazol-1-yl)-acetophenon-(3,3-dimethyl-2-oxo-indolin-6-hydrazon) als Rohprodukt | 23 | >340 Methanol |
| 3-Methyl-6-(4-pyridyl)-benzo[1,2-b:5,4-b']dipyrrol-2(1H,3H,7H)-on aus 4-Acetylpyridin-(3-methyl-2-oxo-indolin-6-hydrazon), Schmp. 267–70°C | 7.5 | >330 Methanol |
| 3-Methyl-6-(3-pyridyl)-benzo[1,2-b:5,4-b']dipyrrol-2(1H,3H,7H)-on aus 3-Acetylpyridin-(3-methyl-2-oxo-indolin-6-hydrazon) als Rohprodukt | 14 | 330–31 Methanol |

Beispiel 2

3,3-Dimethyl-6-(4-cyan-phenyl)-benzo[1,2-b:5,4-b']dipyrrol-2(1H,3H,7H)-on x 1.5 mol $CH_2Cl_2$

Analog Beispiel 1 erhaelt man aus 4.4 g (13.8 mmol) 4-Cyan-acetophenon-(3,3-dimethyl-2-oxo-indolin-6-hydrazon)(Schmp.242° die Titelverbindung. Die Reinigung von 3.7 g Rohprodukt erfolgte durch Saeulenchromatographie ueber Kieselgel (Laufmittel: Methylenchlorid/$CH_3OH$ 95:5). Ausbeute: 0.25 g (6 %), Schmp. >310°C.

Analog Beispiel 2 erhaelt man:

| Bezeichnung | Ausbeute [%] | Schmp. [°C] Lsg.-mittel |
|---|---|---|
| 3,3,6-Trimethyl-benzo[1,2-b:5,4-b']dipyrrol-2(1H,3H,7H)-on aus Aceton-(3,3-dimethyl-2-oxo-indolin-6-hydrazon), Schmp. 150°C | 12.5 | 255 Methanol |
| 3,3,5-Trimethyl-6-(4-pyridyl)-benzo[1,2-b:5,4-b']dipyrrol-2 (1H,3H,7H)-on aus 4-Propionylpyridin-(3,3-dimethyl-2-oxo-indolin-6-hydrazon), Schmp. >300°C | 17.9 | >300°C Methanol |

Beispiel 3

3,3-Dimethyl-6-(2-thienyl)-benzo[1,2-b:5,4-b']dipyrrol-2(1H,3H,7H)-on

Analog Beispiel 1 wurden 6.8 g (38 mmol) 6-Amino-3,3-dimethyl-indolin-2-on diazotiert und reduziert. In die Loesung wurden 10.9 g (86 mmol) 2-Acetyl-thiophen zugetropft, und das abgeschiedene Oel wurde

in Methylenchlorid aufgenommen, getrocknet und eingedampft. Man erhaelt als Rohprodukt 11.2 g 2-Acetylthiophen-(3,3-dimethyl-2-oxo-indolin-6-hydrazon, welches ohne Reinigung weiter umgesetzt wurde.

11 g des Rueckstandes wurden analog Beispiel 1 mit 50 g Polyphosphorsaeure behandelt. Die Reinigung erfolgte durch Saeulenchromatographie (Laufmittel: Methylenchlorid/Essigester 5:1).
Ausbeute: 0.6 g (5.6 %), Schmp.265-70°C (Ethanol) .

Beispiel 4

3,3-Dimethyl-6-phenyl-benzo[1,2-b:5,4-b']dipyrrol-2(1H,3H,7H)-on x 0.5 mol $H_2O$ x 0.14 mol Essigester

Analog Beispiel 1 wurden 3.06 g (17.4 mmol) 6-Amino-3,3-dimethyl-indolin-2-on diazotiert und reduziert. In die Loesung wurden 0.6 g (5 mmol) Acetophenon zugetropft und 3 Stunden bei 25°C weitergeruehrt. Es wurde vom abgeschiedenen Oel abgetrennt, das Oel mit Wasser durchgearbeitet und anschließend mit Heptan ausgekocht. Das Rohprodukt, Acetophenon-(3,3-dimethyl-2-oxo-indolin-6-hydrazon), wurden ohne weitere Reinigung nach Beispiel 1 cyclisiert und nach Beispiel 2 ueber Kieselgel (Laufmittel:Essigester) gereinigt. Man erhaelt 0.16 g (11.6 % bezogen auf Acetophenon) der Titelverbindung vom Schmp. 270-273°C.

Analog Beispiel 4 erhaelt man:

| Bezeichnung | Ausbeute [%] | Schmp. [°C] |
|---|---|---|
| 3,3-Dimethyl-6-(4-methoxy-phenyl)benzo-[1,2-b:5,4-b']dipyrrol-2 (1H,3H,7H)-on x 0.75 mol $H_2O$ aus 4-Methoxy-acetophenon-(3,3-dimethyl-2-oxo-indolin-6-hydrazon) | 3 | 273-76 |

| Bezeichnung | Ausbeute [%] | Schmp. [°C] Lsg.-mittel |
|---|---|---|
| 3,3-Dimethyl-6-(4-methyl-phenyl)benzo[1,2-b:5,4-b']dipyrrol-2(1H,3H,7H)-on x 0.5 mol $H_2O$ x 0.17 mol Essigester aus 4-Methyl-acetophenon-(3,3-dimethyl-2-oxo-indolin-6-hydrazon) | 8.6 | 278–81 |
| 3,3-Dimethyl-6-(4-chlor-phenyl)benzo-[1,2-b:5,4-b']dipyrrol-2(1H,3H,7H)-on x 1.5 mol $H_2O$ x 0.2 mol Essigester aus 4-Chlor-acetophenon-(3,3-dimethyl-2-oxo-indolin-6-hydrazon) | 5.2 | 261–63 |
| 3,3-Dimethyl-6-(2-hydroxy-phenyl)benzo[1,2-b:5,4-b']dipyrrol-2(1H,3H,7H)-on aus 2-Hydroxy-acetophenon-(3,3-dimethyl-2-oxo-indolin-6-hydrazon) | 0.3 | 250–55 |

Beispiel 5

3,3-Dimethyl-6-ethoxycarbonyl-benzo[1,2-b:5,4-b']dipyrrol-2(1H,3H,7H)-on

6 g (34 mmol) 6-Amino-3,3-dimethyl-indolin-2-on werden in 160 ml 2 N HCl geloest und unterhalb von 0°C 2.51 g (36.4 mmol) Natriumnitrit, geloest in 20 ml Wasser, zugetropft. Nach 15 Minuten werden bei der gleichen Temperatur 23.59 g (104 mmol) Zinn(II)chlorid Dihydrat, geloest in 70 ml 2 N HCl, zugetropft. Nach 30 Minuten wurde die Loesung bei 0°C mit 5.58 g (48 mmol) Brenztraubensaeureethylester versetzt und 3 Stunden bei 25°C weitergeruehrt. Der erhaltene Rueckstand wurde abgesaugt, nochmals in Wasser suspendiert und mit waessrigem Ammoniak neutralisiert und abgesaugt. Man erhaelt als Rohprodukt 11.3 g Brenztraubensaeureethylester-(3,3-dimethyl-2-oxo-indolin-6-hydrazon) (Schmp. 284-90°C), welcher ohne Reinigung weiter umgesetzt wurde.

11.3 g des obigen Rohproduktes wurden analog Beispiel 1 in Polyphosphorsaeure bei 100°C cyclisiert. Die Reinigung erfolgte durch Saeulenchromatographie an Kieselgel (Laufmittel: Methylenchlorid/Ethanol 95:5).
Ausbeute: 0.45 g (4.9 % bezogen auf eingesetztes Amin), Schmp. 313-15°C (Ethanol)

Beispiel 6

3,3-Dimethyl-6-carboxy-benzo[1,2-b:5,4-b']dipyrrol-2(1H,3H,7H)-on x 1 mol Ethanol

0.5 g (1.8 mmol) des in Beispiel 5 erhaltenen Esters wurden in 5 ml 2 N Natronlauge und 2.5 ml Ethanol eine Stunde bei 60°C geruehrt. Das Ethanol wurde anschließend abdestilliert, die Loesung mit Kohle behandelt und mit 2 N Salzsaeure angesaeuert. Der Rueckstand wurde aus Ethanol/Methylenchlorid umkristallisiert.
Ausbeute: 82 %, Schmp. 304°C.

Beispiel 7

3,3-Dimethyl-6-aminocarbonyl-benzo[1,2-b:5,4-b']dipyrrol-2(1H,3H,7H)-on x 0.3 mol Ethanol

1.6 g (6.55 mmol) der in Beispiel 6 erhaltenen Carbonsaeure wurden 3 Stunden in 16 ml Thionylchlorid bei 60°C geruehrt. Das ueberschuessige Thionylchlorid wurde anschließend abdestilliert und der Rueckstand unter Kuehlung mit 20 ml konzentriertem Ammoniak versetzt. Nach 24 Stunden bei 25°C wurde der Niederschlag abgesaugt und aus Ethanol umkristallisiert.
Ausbeute: 0.18 g (11 %), Schmp. 294-96°C.

Beispiel 8

3,3-Dimethyl-benzo[1,2-b:5,4-b']dipyrrol-2,6(1H,3H,5H,7H)-dion x 0.5 mol Isopropanol x 0.75 mol $H_2O$

a) 5.0 g (27 mmol) 6-Amino-oxindol Hydrochlorid (Helv. Chim. Acta 20, 373, 1937) wurden in 80 ml $CH_2Cl_2$ suspendiert, mit 6.45 g (63 mmol) Triethylamin versetzt und bei 5°C 6.8 g (29 mmol) α-Bromisobuttersaeurebromid zugetropft. Nach dreistuendigem Ruehren bei 25°C wurde das Loesungsmittel abdestilliert, der Rueckstand mit Wasser durchgearbeitet und abgesaugt. Man erhaelt 4.2g (52.4 %) α-Brom-N-(2-oxo-indolin-6-yl)isobuttersaeureamid von Schmp. 211-12°C.
b) 3.2 g (10.8 mmol) des obigen Amids wurden mit 7.1 g $AlCl_3$ gut vermischt und unter Ruehren 5 Stunden auf 160°C erhitzt. Der abgekuehlte Rueckstand wurde mit Wasser und wenig 2 N-Salzsaeure durchgearbeitet und abgesaugt. Der Niederschlag wurde ueber Kieselgel (Laufmittel: Methylenchlorid/Methanol 96:4) gereinigt.
Ausbeute: 0.27 g (11.7 %), Schmp. 280-82°C (Isopropanol)

Beispiel 9

6'-(4-Pyridyl)-spiro(cyclopentan-1,3'-benzo[1',2'-b:5',4'-b']dipyrrol)-2'(1'H,3'H,7'H)-on

4 g (20 mmol) 6'-Amino-spiro[cyclopentan-1,3'-indolin]-2'-on wurden in 50 ml konzentrierte Salzsaeure eingetragen, auf 0°C gekuehlt und 1.5 g (21.5 mmol) Natriumnitrit, geloest in 5ml Wasser, zugetropft. Nach 15 Minuten werden bei 0°C 13.5 g (60 mmol) Zinn(II)chlorid Dihydrat, geloest in 10 ml konzentrierter Salzsaeure, zugetropft. Nach 2 Stunden bei 25°C werden 2.2 g (18 mmol) 4-Acetyl-pyridin zugegeben und der Niederschlag nach 3 Stunden abgesaugt und mit wenig Wasser gewaschen. Man erhaelt als Rohprodukt 6.7 g 4-Acetyl-pyridin-(2'-oxo-spiro[cyclopentan-1,3'-indolin]-6'-hydrazon-Hydrochlorid, welches ohne weitere Reinigung weiter umgesetzt wurde.
6.7 g des Rohproduktes wurden unter Ruehren in 70 ml Polyphosphorsaeure bei 120°C langsam eingetragen. Nach 2 Stunden bei dieser Temperatur wurde auf Eis gegossen, mit waessrigem Ammoniak neutralisiert und abgesaugt. Die Reinigung erfolgte durch Saeulenchromatographie an Kieselgel (Laufmittel: Methylenchlorid / mit Ammoniak gesaettigtes Methanol 20:1). Ausbeute: 0.7 g (11.5 % bezogen auf eingesetztes Amin), Schmp. 326-29°C (Ethanol).

Beispiel 10

3,3,6-Trimethyl-5-phenyl-benzo[1,2-b:5,4-b']dipyrrol-2(1H,3H,7H)-on

Analog Beispiel 5 wurden 4 g (22.7 mmol) 6-Amino-3,3-dimethyl-indolin-2-on diazotiert und reduziert. Anschließend wurden 3 g (22.3 mmol) Phenylaceton zugesetzt und 18 Stunden bei 25°C weitergeruehrt. Das ausgeschiedene Oel wurde mehrmals mit Methylenchlorid ausgeschuettelt, eingeengt und der Rueckstand mit Cyclohexan durchgearbeitet und abgesaugt. Der feste Rueckstand wurde nochmals in

Methylenchlorid geloest, zweimal mit Natriumhydrogencarbonat-Loesung und einmal mit Wasser gewaschen, mit Natriumsulfat unter Zusatz von Kohle getrocknet und aus Methylenchlorid kristallisiert.
Ausbeute: 1.25 g (19 %), Schmp. 233-35°C.

## Patentansprüche

1. Verbindungen der Formel I

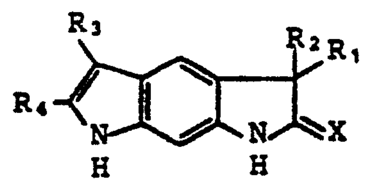

(I),

in welcher
$R_1$ ein Wasserstoffatom, eine $C_1$-$C_6$-Alkyl-, $C_2$-$C_6$-Alkenyl- oder eine $C_3$-$C_7$-Cycloalkylgruppe bedeutet,
$R_2$ ein Wasserstoffatom, eine $C_1$-$C_6$-Alkyl-, $C_2$-$C_6$-Alkenyl- oder Cyangruppe, eine durch eine Hydroxy-, Alkyl-, Alkoxy-, Amino-, Alkylamino-, Dialkylamino- oder Hydrazinogruppe substituierte Carbonylgruppe oder mit $R_1$ zusammen eine $C_3$-$C_7$-Cycloalkylengruppe darstellt oder $R_1$ und $R_2$ zusammen eine $C_1$-$C_6$-Alkyliden- oder $C_3$-$C_7$-Cycloalkylidengruppe bilden, wobei die vorgenannten Alkyl- oder Alkoxyteile 1–6 C-Atome enthalten
$R_3$ ein Wasserstoffatom, eine $C_1$-$C_7$-Alkyl-, $C_2$-$C_7$-Alkenyl-, $C_3$-$C_7$-Cycloalkyl-, $C_3$-$C_7$-Cycloalkenyl-, Cyan-, Alkylcarbonyl-, Alkoxycarbonyl-, Carboxy, Aminocarbonyl-, Alkylaminocarbonyl-, Dialkylaminocarbonyl- oder Arylgruppe bedeutet, wobei die vorgenannten Alkyl- oder Alkoxyteile 1–7 C-Atome enthalten
$R_4$ ein Wasserstoffatom, eine $C_1$-$C_7$-Alkyl-, Trihalogenmethyl-, $C_3$-$C_7$-Cycloalkyl-, Hydroxy-, Cyan-, Carboxy-, Alkoxycarbonyl-, Alkylcarbonyl-, Aminocarbonyl-, Alkylaminocarbonyl- oder Dialkylaminocarbonylgruppe bedeutet, wobei die vorgenannten Alkyl- oder Alkoxyteile 1–7 C-Atome enthalten, oder einen heterocyclischen Fuenfring mit 1-4 Heteroatomen oder einen heterocyclischen Sechsring mit 1-5 Heteroatomen darstellt, wobei die Heteroatome der vorgenannten Fuenf- und Sechsringe gleich oder verschieden sein koennen und Stickstoff, Sauerstoff oder Schwefel bedeuten und gegebenenfalls an einem oder mehreren Stickstoffatomen ein Sauerstoffatom tragen koennen, und die vorgenannten Fuenf- und Sechsringe gegebenenfalls durch eine oder mehrere $C_1$-$C_6$-Alkyl-, $C_1$-$C_6$-Alkoxy-, $C_1$-$C_6$-Alkylmercapto-, Hydroxy-, Nitro-, Amino-, Halogen- oder Cyangruppen substituiert sein koennen,
oder einen Phenylring der allgemeinen Formel II darstellt,

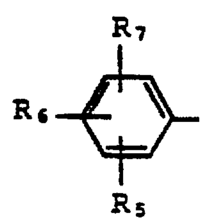

(II),

wobei $R_5$, $R_6$, $R_7$ gleich oder verschieden sein koennen und jeweils Wasserstoff, eine Alkansulfonyloxy-, Trifluormethansulfonyloxy-, Alkansulfonylamino-, Trifluormethansulfonylamino-, N-Alkyl-alkansulfonylamino-, N-Alkyl-trifluormethansulfonylamino-, Alkylsulfenylmethyl-, Alkylsulfinylmethyl- oder Alkylsulfonylmethylgruppe, eine durch eine Hydroxy-, Alkoxy-, Amino-, Alkylamino- oder Dialkylaminogruppe substituierte Carbonylgruppe, eine durch eine Amino-, Alkylamino-, Dialkylamino-, Morpholino-, Pyrrolidino, Piperidino- oder Hexamethyleniminogruppe substituierte Sulfonylgruppe, eine Alkylcarbonylamino-, Aminocarbonylamino- oder Alkylaminocarbonylaminogruppe, eine Alkylmercapto-, Alkylsulfinyl- oder
Alkylsulfonylgruppe, eine Nitro-, Halogen-, Amino-, Hydroxy-, Alkyl-, Alkoxy-, Alkenyloxy-, Alkinyloxy-, Cyanalkyloxy-, Carboxyalkyloxy-, Alkoxycarbonylalkyloxy-, Dialkylamino-, 1-Imidazolyl-, Trifluormethyl- oder Cyangruppe sein koennen, wobei die vorgenannten Alkyl-, Alkenyl oder Alkoxyteile 1–5 C-Atome enthalten,
X ein Sauerstoff- oder Schwefelatom darstellt,
deren Tautomere und deren physiologisch verträgliche Salze anorganischer und organischer Säuren

2. Verbindungen der Formel I gemäß Anspruch 1,
in der
$R_1$ und $R_2$ gleich sind und die Methyl- oder Ethylgruppe darstellen,

$R_1$ und $R_2$ verschieden sind und Wasserstoff, die Methyl-, Ethyl-, Isopropyl-, Cyclopentyl-, Cyan-, Acetyl-, Methoxy-, carbonyl-, Ethoxycarbonyl-, Aminocarbonyl- und Hydrazinocarbonylgruppe bedeuten,
$R_1$ und $R_2$ einen Spirocyclopentylring darstellen, wenn $R_1$ und $R_2$ mit dem C-Atom, an das sie gebunden sind, einen Cycloalkylring bilden,
$R_3$ Wasserstoff, die Methyl-, Ethyl-, Isopropyl-, n-Butyl-, Allyl-, Cyclohexyl-, Cyclopentenyl-, Cyan-, Ethoxycarbonyl- oder Phenylgruppe bedeutet,
$R_4$ die Methyl-, Ethyl-, Isopropyl-, Trifluormethyl-, Cyclopentyl-, Hydroxy-, Cyan-, Acetyl-, Carboxy-, Ethoxycarbonyl-, Aminocarbonyl-, Methylaminocarbonyl- oder Dimethylaminocarbonylgruppe bedeutet oder
$R_4$ den Pyrrol-, Furan-, Thiophen, Pyrazol-, Imidazol-, Thiazol-, Oxazol-, Triazol-, Tetrazol-, Thiadiazol-, Oxadiazol-, Pyridin-, N-Oxy-pyridin-, Pyrazin-, N,N-Dioxy-pyrazin-, Pyrimidin-, N,N-Dioxypyrimidin-, Pyridazin-, Oxazin-, Thiazin-, Triazin- oder Tetrazinrest darstellt, wobei die aufgeführten Reste durch Methyl-, Ethyl-, Methoxy-, Ethoxy-, Methylmercapto-, Ethylmercapto-Gruppen und Chlor substituiert sein können,
oder den Phenylrest der allgemeinen Formel II, in der
$R_5$ ein Wasserstoff, die Methansulfonyloxy-, Trifluormethansulfonyloxy-, Methansulfonylamino-, Trifluormethansulfonylamino-, Methansulfonyl-methylamino-, Trifluormethansulfonylmethylamino-, Methylsulfenylmethyl-, Methylsulfinylmethyl-, Methylsulfonylmethyl-, Aminocarbonyl-, Aminosulfonyl-, Methylaminosulfonyl-, Dimethylaminosulfonyl-, Acetylamino-, Methylmercapto-, Methylsulfonyl-, Hydroxy-, Methyl-, Methoxy-, Propargyloxy-, Cyanmethyloxy-, Methoxycarbonylmethyloxy-, Cyan-, Chlor-, Nitro-, Amino-, Dimethylamino-,
Trifluormethyl- oder die 1-Imidazolylgruppe,
$R_6$ Wasserstoff, die Methyl-, Methoxy-, Dimethylamino- oder Chlorgruppe bedeutet,
$R_7$ Wasserstoff oder die Methoxygruppe ist und
X fuer ein Sauerstoffatom steht,
deren Tautomere und deren physiologisch verträgliche Salze anorganischer und organischer Säuren.

3. Verbindungen der Formel I gemäß Anspruch 1 oder 2, in der
$R_1$ die Methyl- oder Ethylgruppe,
$R_2$ Wasserstoff, die Methyl- oder Ethylgruppe oder mit $R_1$ zusammen den Cyclopentylring bedeuten,
$R_3$ Wasserstoff, die Methyl-, Ethyl- oder Phenylgruppe darstellt,
$R_4$ die Methyl-, Ethyl-, Hydroxy-, Carboxy-, Ethoxycarbonyl- oder Aminocarbonylgruppe oder den Thiophen-, Pyridin- oder Pyridazinrest oder die Phenylgruppe, die gegebenenfalls durch Hydroxy, Methoxy, Cyan, Chlor, Methyl oder 1-Imidazolyl substituiert sein kann, bedeutet und
X für ein Sauerstoffatom steht,
deren Tautomere und deren physiologisch verträgliche Salze anorganische und organische Säuren.

4. Verfahren zur Herstellung von Verbindungen der Formel I

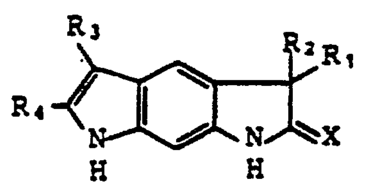

(I),

in welcher
$R_1$ ein Wasserstoffatom, eine $C_1$-$C_6$-Alkyl-, $C_2$-$C_6$-Alkenyl- oder eine $C_3$-$C_7$-Cycloalkylgruppe bedeutet,
$R_2$ ein Wasserstoffatom, eine $C_1$-$C_6$-Alkyl-, $C_2$-$C_6$-Alkenyl- oder Cyangruppe,
eine durch eine Hydroxy-, Alkyl-, Alkoxy-, Amino-, Alkylamino-, Dialkylamino- oder Hydrazinogruppe substituierte Carbonylgruppe oder mit $R_1$ zusammen eine $C_3$-$C_7$-Cycloalkylengruppe darstellt oder $R_1$ und $R_2$ zusammen eine $C_1$-$C_6$-Alkyliden- oder $C_3$-$C_7$-Cycloalkylidengruppe bilden, wobei die vorgenannten Alkyl- oder Alkoxyteile 1–6 C-Atome enthalten,
$R_3$ ein Wasserstoffatom, eine $C_1$-$C_7$-Alkyl-, $C_2$-$C_7$-Alkenyl-, $C_3$-$C_7$-Cycloalkyl-, $C_3$-$C_7$-Cycloalkenyl-, Cyan-, Alkylcarbonyl-, Alkoxycarbonyl-, Carboxy, Aminocarbonyl-, Alkylaminocarbonyl-, Dialkylaminocarbonyl- oder Arylgruppe bedeutet, wobei die vorgenannten Alkyl- oder Alkoxyteile 1–7 C-Atome enthalten,
$R_4$ ein Wasserstoffatom, eine $C_1$-$C_7$-Alkyl-, Trihalogenmethyl-, $C_3$-$C_7$-Cycloalkyl-, Hydroxy-, Cyan-, Carboxy-, Alkoxycarbonyl-, Alkylcarbonyl-, Aminocarbonyl-, Alkylaminocarbonyl- oder Dialkylaminocarbonylgruppe bedeutet, wobei die vorgenannten Alkyl- oder Alkoxyteile 1–7 C-Atome enthalten, oder
einen heterocyclischen Fuenfring mit 1-4 Heteroatomen oder einen heterocyclischen Sechsring mit 1-5 Heteroatomen darstellt, wobei die Heteroatome der vorgenannten Fuenf- und Sechsringe gleich oder verschieden sein koennen und Stickstoff, Sauerstoff oder Schwefel bedeuten und gegebenenfalls an einem oder mehreren Stickstoffatomen ein Sauerstoffatom tragen koennen, und die vorgenannten

Fuenf- und Sechsringe gegebenenfalls durch eine oder mehrere $C_1$-$C_6$-Alkyl-, $C_1$-$C_6$-Alkoxy-, $C_1$-$C_6$-Alkylmercapto-, Hydroxy-, Nitro-, Amino-, Halogen- oder Cyangruppen substituiert sein koennen, oder einen Phenylring der allgemeinen Formel II darstellt,

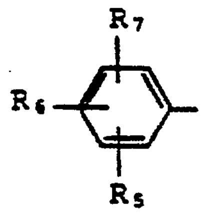

(II),

wobei $R_5$, $R_6$, $R_7$ gleich oder verschieden sein koennen und jeweils Wasserstoff, eine Alkansulfonyloxy-, Trifluormethansulfonyloxy-, Alkansulfonylamino-, Trifluormethansulfonylamino-, N-Alkyl-alkansulfonylamino-, N-Alkyltrifluormethansulfonylamino-, Alkylsulfenylmethyl-, Alkylsulfinylmethyl- oder Alkylsulfonylmethylgruppe, eine durch eine Hydroxy-, Alkoxy-, Amino-, Alkylamino- oder Dialkylaminogruppe substituierte Carbonylgruppe, eine durch eine Amino-, Alkylamino-, Dialkylamino-, Morpholino-, Pyrrolidino, Piperidino- oder Hexamethyleniminogruppe substituierte Sulfonylgruppe, eine Alkylcarbonylamino-, Aminocarbonylamino- oder Alkylaminocarbonylaminogruppe, eine Alkylmercapto-, Alkylsulfinyl- oder
Alkylsulfonylgruppe, eine Nitro-, Halogen-, Amino-, Hydroxy-, Alkyl-, Alkoxy-, Alkenyloxy-, Alkinyloxy-, Cyanalkyloxy-, Carboxyalkyloxy-, Alkoxycarbonylalkyloxy-, Dialkylamino-, 1-Imidazolyl-, Trifluormethyl- oder Cyangruppe sein koennen, wobei die vorgenannten Alkyl-, Alkenyl oder Alkoxyteile 1–5 C-Atome enthalten,
X ein Sauerstoff- oder Schwefelatom darstellt,
deren Tautomere und deren physiologisch verträgliche Salze
dadurch gekennzeichnet, daß man in an sich bekannter Weise
a) Verbindungen der Formel VI

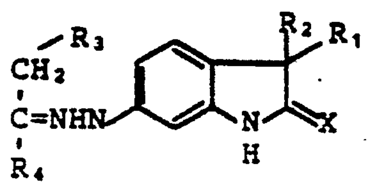

(VI),

in der $R_1$, $R_2$, $R_3$, $R_4$ und X die angegebenen Bedeutungen haben,
in einer Fischer-Indol-Synthese cyclisiert
oder
b) Verbindungen der Formel III

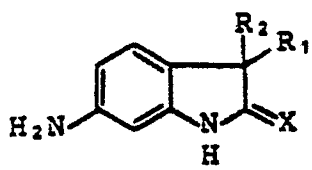

(III)

bzw. der Formel XI

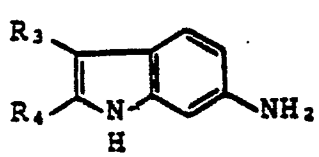

(XI),

in denen $R_1$, $R_2$, $R_3$, $R_4$ und X die angegebenen Bedeutungen haben,
mit Bisulfit-Additions-Verbindungen von entsprechenden Ketonen (Hinsberg-Synthese) oder über ein entsprechendes Hydrazid (Brunner-Synthese) oder über ein entsprechendes Amid (Stollé-Synthese) cyclisiert
und anschließend gewünschtenfalls erhaltene Verbindungen der Formel I in andere Verbindungen der Formel I umwandelt sowie gegebenenfalls die Verbindungen in pharmakologisch verträgliche Salze überführt.

5. Verfahren zur Herstellung von Verbindungen der Formel I gemäß Anspruch 4, in der
$R_1$ und $R_2$ gleich sind und die Methyl- oder Ethylgruppe darstellen,
$R_1$ und $R_2$ verschieden sind und Wasserstoff, die Methyl-, Ethyl-, Isopropyl-, Cyclopentyl-, Cyan-, Acetyl-, Methoxycarbonyl-, Ethoxycarbonyl-, Aminocarbonyl- und Hydrazinocarbonylgruppe bedeuten,
$R_1$ und $R_2$ einen Spirocyclopentylring darstellen, wenn $R_1$ und $R_2$ mit dem C-Atom, an das sie gebunden sind, einen Cycloalkylring bilden,
$R_3$ Wasserstoff, die Methyl-, Ethyl-, Isopropyl-, n-Butyl-, Allyl-, Cyclohexyl-, Cyclopentenyl-, Cyan-, Ethoxycarbonyl- oder Phenylgruppe bedeutet,
$R_4$ die Methyl-, Ethyl-, Isopropyl-, Trifluormethyl-, Cyclopentyl-, Hydroxy-, Cyan-, Acetyl-, Carboxy-, Ethoxycarbonyl-, Aminocarbonyl-, Methylaminocarbonyl- oder Dimethylaminocarbonylgruppe bedeutet oder
$R_4$ den Pyrrol-, Furan-, Thiophen-, Pyrazol-, Imidazol-, Thiazol-, Oxazol-, Triazol-, Tetrazol-, Thiadiazol-, Oxadiazol-, Pyridin-, N-Oxy-pyridin-, Pyrazin-, N,N-Dioxy-pyrazin-, Pyrimidin-, N,N-Dioxypyrimidin-, Pyridazin-, Oxazin-, Thiazin-, Triazin- oder Tetrazinrest darstellt, wobei die aufgeführten Reste durch Methyl-, Ethyl-, Methoxy-, Ethoxy-, Methylmercapto-, Ethylmercapto-Gruppen und Chlor substituiert sein können,
oder den Phenylrest der allgemeinen Formel II, in der
$R_5$ ein Wasserstoff, die Methansulfonyloxy-, Trifluormethansulfonyloxy-, Methansulfonylamino-, Trifluormethansulfonylamino-, Methansulfonyl-methylamino-, Trifluormethansulfonylethylamino-, Methylsulfenylmethyl-, Methylsulfinylmethyl-, ethylsulfonylmethyl-, Aminocarbonyl-, Aminosulfonyl-, Methylaminosulfonyl-, Dimethylaminosulfonyl-, Acetylamino-, Methylmercapto-, Methylsulfonyl-, Hydroxy-, Methyl-, Methoxy-, Propargyloxy-, Cyanmethyloxy-, Methoxycarbonylmethyloxy-, Cyan-, Chlor-, Nitro-, Amino-, Dimethylamino-,
Trifluormethyl- oder die 1-Imidazolylgruppe,
$R_6$ Wasserstoff, die Methyl-, Methoxy-, Dimethylamino- oder Chlorgruppe bedeutet,
$R_7$ Wasserstoff oder die Methoxygruppe ist und
X fuer ein Sauerstoffatom steht,
deren Tautomere und deren physiologisch verträgliche Salze.

6. Verfahren zur Herstellung von Verbindungen der Formel I gemäß Anspruch 4 in der
$R_1$ die Methyl- oder Ethylgruppe,
$R_2$ Wasserstoff, die Methyl- oder Ethylgruppe oder mit $R_1$ zusammen den Cyclopentylring bedeuten,
$R_3$ Wasserstoff, die Methyl-, Ethyl- oder Phenylgruppe darstellt,
$R_4$ die Methyl-, Ethyl-, Hydroxy-, Carboxy-, Ethoxycarbonyl- oder Aminocarbonylgruppe oder den Thiophen-, Pyridin- oder Pyridazinrest oder die Phenylgruppe, die gegebenenfalls durch Hydroxy, Methoxy, Cyan, Chlor, Methyl oder 1-Imidazolyl substituiert sein kann, bedeutet und
X für ein Sauerstoffatom steht,
deren Tautomere und deren physiologisch verträgliche Salze anorganischer und organischer Säuren

7. Verwendung von Verbindungen gemäß Anspruch 1, 2 oder 3 zur Herstellung von Arzneimitteln für die Behandlung von Herz- und/oder Kreislauferkrankungen.

8. Arzneimittel, enthaltend eine Verbindung gemäß Anspruch 1, 2 oder 3 neben üblichen pharmakologischen Träger- und/oder Hilfsstoffen.

9. Verbindungen der Formel VI

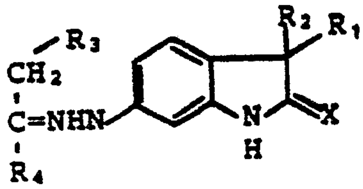

(VI),

in welcher
$R_1$ ein Wasserstoffatom, eine $C_1$-$C_6$-Alkyl-, $C_2$-$C_6$-Alkenyl- oder eine $C_3$-$C_7$-Cycloalkylgruppe bedeutet,
$R_2$ ein Wasserstoffatom, eine $C_1$-$C_6$-Alkyl-, $C_2$-$C_6$-Alkenyl- oder Cyangruppe, eine durch eine Hydroxy-, Alkyl-, Alkoxy-, Amino-, Alkylamino-, Dialkylamino- oder Hydrazinogruppe substituierte Carbonylgruppe oder mit $R_1$ zusammen eine $C_3$-$C_7$-Cycloalkylengruppe darstellt oder $R_1$ und $R_2$ zusammen eine $C_1$-$C_6$-Alkyliden- oder $C_3$-$C_7$-Cycloalkylidengruppe bilden, wobei die vorgenannten Alkyl- oder Alkoxyteile 1–6 C-Atome enthalten,
$R_3$ ein Wasserstoffatom, eine Alkyl-, Alkenyl-, Cycloalkyl-, Cycloalkenyl-, Cyan-, Alkylcarbonyl-, Alkoxycarbonyl-, Carboxy-, Aminocarbonyl-, Alkylaminocarbonyl-, Dialkylaminocarbonyl- oder Arylgruppe bedeutet,
$R_4$ ein Wasserstoffatom, eine $C_1$-$C_7$-Alkyl-, Trihalogenmethyl-, $C_3$-$C_7$-Cycloalkyl-, Hydroxy-, Cyan-,

Carboxy-, Alkoxycarbonyl-, Alkylcarbonyl-, Aminocarbonyl-, Alkylaminocarbonyl- oder Dialkylaminocarbonylgruppe bedeutet, wobei die vorgenannten Alkyl- oder Alkoxyteile 1–7 C-Atome enthalten, oder einen heterocyclischen Fuenfring mit 1-4 Heteroatomen oder einen heterocyclischen Sechsring mit 1-5 Heteroatomen darstellt, wobei die Heteroatome der vorgenannten Fuenf- und Sechsringe gleich oder verschieden sein koennen und Stickstoff, Sauerstoff oder Schwefel bedeuten und gegebenenfalls an einem oder mehreren Stickstoffatomen ein Sauerstoffatom tragen koennen, und die vorgenannten Fuenf- und Sechsringe gegebenenfalls durch eine oder mehrere $C_1$-$C_6$-Alkyl-, $C_1$-$C_6$-Alkoxy-, $C_1$-$C_6$-Alkylmercapto-, Hydroxy-, Nitro-, Amino-, Halogen- oder Cyangruppen substituiert sein koennen,
oder einen Phenylring der allgemeinen Formel II darstellt,

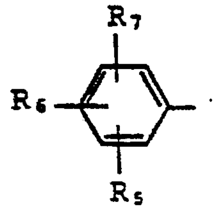

(II),

wobei $R_5$, $R_6$, $R_7$ gleich oder verschieden sein koennen und jeweils Wasserstoff, eine Alkansulfonyloxy-, Trifluormethansulfonyloxy-, Alkansulfonylamino-, Trifluormethansulfonylamino-, N-Alkyl-alkansulfonylamino-, N-Alkyl-trifluormethansulfonylamino-, Alkylsulfenylmethyl-, Alkylsulfinylmethyl- oder Alkylsulfonylmethylgruppe, eine durch eine Hydroxy-, Alkoxy-, Amino-, Alkylamino- oder Dialkylaminogruppe substituierte Carbonylgruppe, eine durch eine Amino-, Alkylamino-, Dialkylamino-, Morpholino-, Pyrrolidino, Piperidino- oder Hexamethyleniminogruppe substituierte Sulfonylgruppe, eine Alkylcarbonylamino-, Aminocarbonylamino- oder Alkylaminocarbonylaminogruppe, eine Alkylmercapto-, Alkylsulfinyl- oder
Alkylsulfonylgruppe, eine Nitro-, Halogen-, Amino-, Hydroxy-, Alkyl-, Alkoxy-, Alkenyloxy-, Alkinyloxy-, Cyanalkyloxy-, Carboxyalkyloxy-, Alkoxycarbonylalkyloxy-, Dialkylamino-, 1-Imidazolyl-, Trifluormethyl- oder Cyangruppe sein koennen, wobei die vorgenannten Alkyl-, Alkenyl- oder Alkoxyteile 1–5 C-Atome enthalten, wobei die vorgenannten Alkyl-, Alkenyl- oder Alkoxyteile 1–5 C-Atome enthalten, und X ein Sauerstoff- oder Schwefelatom darstellt.

10. Verfahren zur Herstellung von Verbindungen der Formel VI

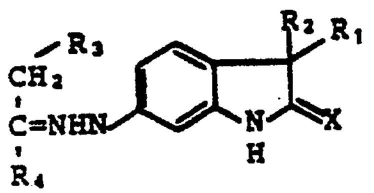

(VI),

in welcher
$R_1$ ein Wasserstoffatom, eine $C_1$-$C_6$-Alkyl-, $C_2$-$C_6$-Alkenyl- oder eine $C_3$-$C_7$-Cycloalkylgruppe bedeutet,
$R_2$ ein Wasserstoffatom, eine $C_1$-$C_6$-Alkyl-, $C_2$-$C_6$-Alkenyl- oder Cyangruppe, eine durch eine Hydroxy-, Alkyl-, Alkoxy-, Amino-, Alkylamino-, Dialkylamino- oder Hydrazinogruppe substituierte Carbonylgruppe oder mit $R_1$ zusammen eine $C_3$-$C_7$-Cycloalkylengruppe darstellt oder $R_1$ und $R_2$ zusammen eine $C_1$-$C_6$-Alkyliden- oder $C_3$-$C_7$-Cycloalkylidengruppe bilden, wobei die vorgenannten Alkyl- oder Alkoxyteile 1–6 C-Atome enthalten,
$R_3$ ein Wasserstoffatom, eine $C_1$-$C_7$-Alkyl-, $C_2$-$C_7$-Alkenyl-, $C_3$-$C_7$-Cycloalkyl-, $C_3$-$C_7$-Cycloalkenyl-, Cyan-, Alkylcarbonyl-, Alkoxycarbonyl-, Carboxy-, Aminocarbonyl-, Alkylaminocarbonyl-, Dialkylaminocarbonyl- oder Arylgruppe bedeutet, wobei die vorgenannten Alkyl- oder Alkoxyteile 1–7 C-Atome enthalten,
$R_4$ ein Wasserstoffatom, eine $C_1$-$C_7$-Alkyl-, Trihalogenmethyl-, $C_3$-$C_7$-Cycloalkyl-, Hydroxy-, Cyan-, Carboxy-, Alkoxycarbonyl-, Alkylcarbonyl-, Aminocarbonyl-, Alkylaminocarbonyl- oder Dialkylaminocarbonylgruppe bedeutet, wobei die vorgenannten Alkyl- oder Alkoxyteile 1–7 C-Atome enthalten, oder einen heterocyclischen Fuenfring mit 1-4 Heteroatomen oder einen heterocyclischen Sechsring mit 1-5 Heteroatomen darstellt, wobei die Heteroatome der vorgenannten Fuenf- und Sechsringe gleich oder verschieden sein koennen und Stickstoff, Sauerstoff oder Schwefel bedeuten und gegebenenfalls an einem oder mehreren Stickstoffatomen ein Sauerstoffatom tragen koennen, und die vorgenannten Fuenf- und Sechsringe gegebenenfalls durch eine oder mehrere Alkyl-, Alkoxy-, Alkylmercapto-, Hydroxy-, Nitro-, Amino-, Halogen- oder Cyangruppen substituiert sein koennen,
oder einen Phenylring der allgemeinen Formel II darstellt,

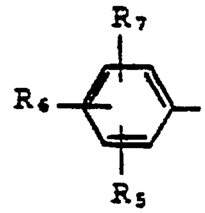

(II),

wobei $R_5$, $R_6$, $R_7$ gleich oder verschieden sein koennen und jeweils Wasserstoff, eine Alkansulfonyloxy-, Trifluormethansulfonyloxy-, Alkansulfonylamino-, Trifluormethansulfonylamino-, N-Alkyl-alkansulfonylamino-, N-Alkyl-trifluormethansulfonylamino-, Alkylsulfenylmethyl-, Alkylsulfinylmethyl- oder Alkylsulfonylmethylgruppe, eine durch eine Hydroxy-, Alkoxy-, Amino-, Alkylamino- oder Dialkylaminogruppe substituierte Carbonylgruppe, eine durch eine Amino-, Alkylamino-, Dialkylamino-, Morpholino-, Pyrrolidino, Piperidino- oder Hexamethyleniminogruppe substituierte Sulfonylgruppe, eine Alkylcarbonylamino-, Aminocarbonylamino- oder Alkylaminocarbonylaminogruppe, eine Alkylmercapto-, Alkylsulfinyl- oder
Alkylsulfonylgruppe, eine Nitro-, Halogen-, Amino-, Hydroxy-, Alkyl-, Alkoxy-, Alkenyloxy-, Alkinyloxy-, Cyanalkyloxy-, Carboxyalkyloxy-, Alkoxycarbonylalkyloxy-, Dialkylamino-, 1-Imidazolyl-, Trifluormethyl- oder Cyangruppe sein koennen, wobei die vorgenannten Alkyl-, Alkenyl oder Alkoxyteile 1–5 C-Atome enthalten, und
X ein Sauerstoff- oder Schwefelatom darstellt,
dadurch gekennzeichnet, daß man nach an sich bekannten Methoden Amine der Formel III

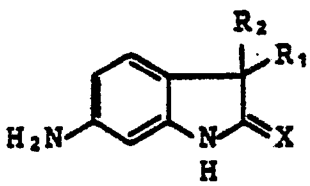

(III),

in der $R_1$, $R_2$ und X die angegebenen Bedeutungen haben,
A) diazotiert, anschließend entweder
a) zu den Hydrazinen der Formel IV

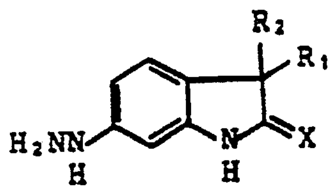

(IV),

in der $R_1$, $R_2$ und X die angegebenen Bedeutungen haben, reduziert und mit Verbindungen der Formel V

$$R_4COCH_2R_3 \text{ (V)},$$

in der $R_3$ und $R_4$ die angegebenen Bedeutungen haben, umsetzt
oder
b) in einer Japp-Klingemann-Reaktion mit einer Verbindung der Formel VII

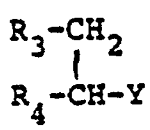

(VII),

in der $R_3$ und $R_4$ die angegebenen Bedeutungen haben und Y einen aktivierenden Rest darstellt, umsetzt und anschließend verseift
oder
B) mit Halogenessigestern X

$$Z\text{-}CH_2\text{-}COOEt \text{ (X)},$$

in denen Z Halogen bedeutet, umsetzt, anschließend verseift und zu den Verbindungen VIII

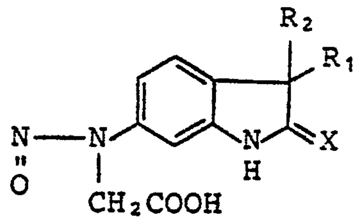

(VIII),

in denen $R_1$, $R_2$ und X die oben angegebene Bedeutung haben, nitrosiert, diese mit wasserentziehenden Reagenzien zu den Sydnonen IX

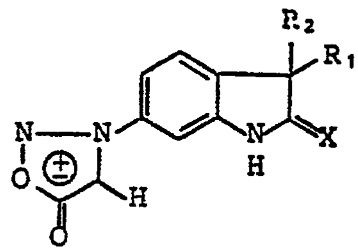

(IX),

umsetzt, die mit den Ketonen V

$R_4COCH_2R_3$ (V),

in denen $R_3$ und $R_4$ die oben angegebenen Bedeutungen haben, in Verbindungen der Formel VI umgewandelt werden.

**Claims**

1. Compounds of the formula I

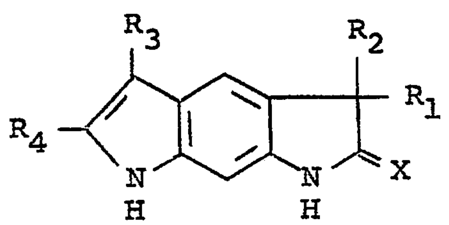

(I)

in which $R_1$ signifies a hydrogen atom, a $C_1$–$C_6$-alkyl, $C_2$–$C_6$-alkenyl or a $C_3$–$C_7$-cycloalkyl group, $R_2$ represents a hydrogen atom, a $C_1$–$C_6$-alkyl, $C_2$–$C_6$-alkenyl or cyano group, a carbonyl group substituted by a hydroxyl, alkyl, alkoxy, amino, alkylamino, dialkylamino or hydrazino group or together with $R_1$ a $C_3$–$C_7$-cycloalkylene group or $R_1$ and $R_2$ together form a $C_1$–$C_6$-alkylidene or $C_3$–$C_7$-cycloalkylidene radical, whereby the above-mentioned alkyl or alkoxy moieties contain 1–6 C-atoms, $R_3$ signifies a hydrogen atom, a $C_1$–$C_7$-alkyl, $C_2$–$C_7$-alkenyl, $C_3$–$C_7$-cycloalkyl, $C_3$–$C_7$-cycloalkenyl, cyano, alkylcarbonyl, alkoxycarbonyl, carboxyl, aminocarbonyl, alkylaminocarbonyl, dialkylaminocarbonyl or aryl group, whereby the above-mentioned alkyl or alkoxy moieties contain 1–7 C-atoms, $R_4$ signifies a hydrogen atom, a $C_1$–$C_7$-alkyl, trihalogenomethyl, $C_3$–$C_7$-cycloalkyl, hydroxyl, cyano, carboxyl, alkoxycarbonyl, alkylcarbonyl, aminocarbonyl, alkylaminocarbonyl or dialkylaminocarbonyl group, whereby the above-mentioned alkyl or alkoxy moieties contain 1–7 C-atoms, or a heterocyclic five-membered ring with 1–4 heteroatoms or a heterocyclic six-membered rings with 1–5 heteroatoms, whereby the heteroatoms of the above-mentioned five- and six-membered rings can be the same or different and signify nitrogen, oxygen or sulphur and on one or more nitrogen atoms can carry an oxygen atom and the above-mentioned five- and six-membered rings can optionally be substituted by one or more $C_1$–$C_6$-alkyl, $C_1$–$C_6$-alkoxy, $C_1$–$C_6$-alkylmercapto, hydroxyl, nitro, amino, halogen or cyano groups, or represents a phenyl ring of the general formula II

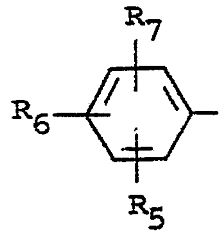

(II)

whereby $R_5$, $R_6$, $R_7$ can be the same or different and in each case can be hydrogen, an alkanesulphonyloxy, trifluoromethanesulphonyloxy, alkanesulphonylamino, trifluoromethanesulphonylamino, N-alkyl-alkanesulphonylamino, N-alkyl-trifluoromethanesulphonylamino, alkylsulphenylmethyl, alkylsulphinylmethyl or alkylsulphonylmethyl group, a carbonyl group substituted by a hydroxyl, alkoxy, amino, alkylamino or dialkylamino group, a sulphonyl group substituted by an amino, alkylamino, dialkylamino, morpholino, pyrrolidino, piperidino or hexamethyleneimino group, an alkylcarbonylamino, aminocarbonylamino or alkylaminocarbonylamino group, an alkylmercapto, alkylsulphinyl or alkylsulphonyl group, a nitro, halogen, amino, hydroxyl, alkyl, alkoxy, alkenyloxy, alkynyloxy, cyanoalkoxy, carboxyalkoxy, alkoxycarbonylalkoxy, dialkylamino, 1-imidazolyl, trifluoromethyl or cyano group, whereby the above-mentioned alkyl, alkenyl or alkoxy moieties contain 1–5 C-atoms, X represents an oxygen or sulphur atom; their tautomers and their physiologically acceptable salts of inorganic and organic acids.

2. Compounds of formula I according to claim 1, in which $R_1$ and $R_2$ are the same and represent the methyl or ethyl group, $R_1$ and $R_2$ are different and signify hydrogen, the methyl, ethyl, isopropyl, cyclopentyl, cyano, acetyl, methoxycarbonyl, ethoxycarbonyl, aminocarbonyl and hydrazinocarbonyl group, $R_1$ and $R_2$ represent a spirocyclopentyl ring when $R_1$ and $R_2$, with the C-atom to which they are attached, form a cycloalkyl ring, $R_3$ signifies hydrogen, the methyl, ethyl, isopropyl, n-butyl, allyl, cyclohexyl, cyclopentenyl, cyano, ethoxycarbonyl or phenyl group, $R_4$ signifies the methyl, ethyl, isopropyl, trifluoromethyl, cyclopentyl, hydroxyl, cyano, acetyl, carboxyl, ethoxycarbonyl, aminocarbonyl, ethylaminocarbonyl or dimethylaminocarbonyl group or $R_4$ represents the pyrrole, furan, thiophene, pyrazole, imidazole, thiazole, oxazole, triazole, tetrazole, thiadiazole, oxadiazole, pyridine, N-oxypyridine, pyrazine, N,N-dioxypyrazine, pyrimidine, N,N-dioxypyrimidine, pyridazine, oxazine, thiazine, thiazine or tetrazine radical, whereby the stated radicals can be substituted by methyl, ethyl, methoxy, ethoxy, methylmercapto, ethylmercapto groups and chlorine or the phenyl group of the general formula II, in which $R_5$ signifies a hydrogen, the methanesulphonyloxy, trifluoromethanesulphonyloxy, methanesulphonylamino, trifluoromethanesulphonylamino, methanesulphonylmethylamino, trifluoromethanesulphonylmethylamino, methylsulphenylmethyl, methylsulphinylmethyl, methylsulphonylmethyl, aminocarbonyl, aminosulphonyl, methylaminosulphonyl, dimethylaminosulphonyl, acetylamino, methylmercapto, methylsulphonyl, hydroxyl, methyl, methoxy, propargyloxy, cyanomethyloxy, methoxycarbonylmethyloxy, cyano, chloro, nitro, amino, dimethylamino, trifluoromethyl or the 1-imidazolyl group, $R_6$ hydrogen, the methyl, methoxy, dimethylamino or chlorine group, $R_7$ is hydrogen or the methoxy group and X stands for an oxygen atom; their tautomers and their physiologically acceptable salts of inorganic and organic acids.

3. Compounds of the formula I according to claim 1 or 2, in which $R_1$ signifies the methyl or ethyl group, $R_2$ hydrogen, the methyl or ethyl group or together with $R_1$ the cyclopentyl ring, $R_3$ represents hydrogen, the methyl, ethyl or phenyl group, $R_4$ signifies the methyl, ethyl, hydroxyl, carboxyl, ethoxycarbonyl or aminocarbonyl group or the thiophene, pyridine or pyridazine radical or the phenyl group which can optionally be substituted by hydroxyl, methoxy, cyano, chlorine, methyl or 1-imidazolyl and X stands for an oxygen atom; their tautomers and their physiologically acceptable salts of inorganic and organic acids.

4. Process for the preparation of compounds of the formula I

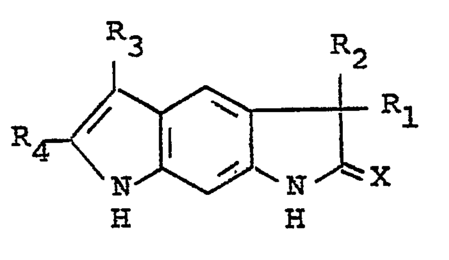

(I)

in which $R_1$ signifies a hydrogen atom, a $C_1$–$C_6$-alkyl, $C_2$–$C_6$-alkenyl or $C_3$–$C_7$-cycloalkyl group, $R_2$ represents a hydrogen atom, a $C_1$–$C_6$-alkyl, $C_2$–$C_6$-alkenyl or cyano group, a carbonyl group substituted by a hydroxyl, alkyl, alkoxy, amino, alkylamino, dialkylamino or hydrazino group or together with $R_1$ a $C_3$–$C_7$-cycloalkylene group or $R_1$ and $R_2$ together form a $C_1$-$C_6$- alkylidene or $C_3$–$C_7$-cycloalkylidene group, whereby the above-mentioned alkyl or alkoxy moieties contain 1–6 C-atoms, $R_3$ signifies a hydrogen at-

om, a $C_1$–$C_7$-alkyl, $C_2$–$C_7$-alkenyl, $C_3$–$C_7$-cycloalkyl, $C_3$–$C_7$-cycloalkenyl, cyano, alkylcarbonyl, alkoxycarbonyl, carboxyl, aminocarbonyl, alkylaminocarbonyl, dialkylaminocarbonyl or aryl group, whereby the above-mentioned alkyl or alkoxy moieties contain 1–7 C-atoms, $R_4$ signifies a hydrogen atom, a $C_1$–$C_7$-alkyl, trihalogenomethyl, $C_3$–$C_7$-cycloalkyl, hydroxyl, cyano, carboxyl, alkoxycarbonyl, alkylcarbonyl, aminocarbonyl, alkylaminocarbonyl or dialkylaminocarbonyl group, whereby the above-mentioned alkyl or alkoxy moieties contain 1–7 C-atoms, or a heterocyclic five-membered ring with 1–4 heteroatoms or a heterocyclic six-membered ring with 1–5 heteroatoms, whereby the heteroatoms of the above-mentioned five- and six-membered rings can be the same or different and signify nitrogen, oxygen or sulphur and on one or more nitrogen atoms can optionally carry an oxygen atom, and the above-mentioned five- and six-membered rings can optionally be substituted by one or more $C_1$–$C_6$-alkyl, $C_1$–$C_6$-alkoxy, $C_1$–$C_6$-alkylmercapto, hydroxyl, nitro, amino, halogen or cyano groups or $R_4$ represents a phenyl ring of the general formula II

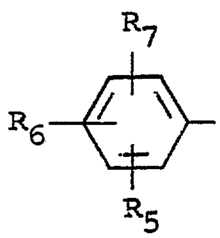

(II)

whereby $R_5$, $R_6$, $R_7$ can be the same or different and in each case can be hydrogen, an alkanesulphonyloxy, trifluoromethanesulphonyloxy, alkanesulphonylamino, trifluoromethanesulphonylamino, N-alkyl-alkanesulphonylamino, N-alkyl-trifluoromethanesulphonylamino, alkylsulphenylmethyl, alkylsulphinylmethyl or alkylsulphonylmethyl group, a carbonyl group substituted by a hydroxyl, alkoxy, amino, alkylamino or dialkylamino group, a sulphonyl group substituted by amino, alkylamino, dialkylamino, morpholino, pyrrolidino, piperidino or hexamethyleneimino group, an alkylcarbonylamino, aminocarbonylamino or alkylaminocarbonylamino group, an alkylmercapto, alkylsulphinyl or alkylsulphonyl group, a nitro, halogen, amino, hydroxyl, alkyl, alkoxy, alkenyloxy, alkynyloxy, cyanoalkyloxy, carboxyalkyloxy, alkoxycarbonylalkyloxy, dialkylamino, 1-imidazolyl, trifluoromethyl or cyano group, whereby the above-mentioned alkyl, alkenyl or alkoxy moieties contain 1–5 C-atoms, X represents an oxygen or sulphur atom, their tautomers and their physiologically acceptable salts, characterised in that, in per se known manner, one

a) cyclises compounds of the formula VI

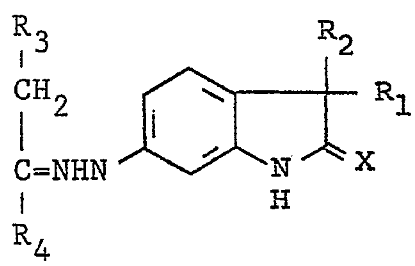

(VI)

in which $R_1$, $R_2$, $R_3$, $R_4$ and X have the given meanings, in a Fischer indole synthesis; or

b) cyclises compounds of the formula III

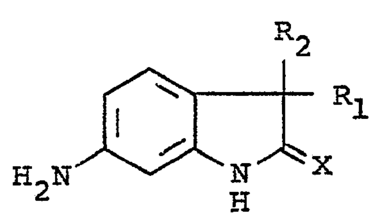

(III)

or of the formula XI

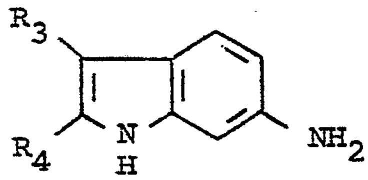

(XI)

in which $R_1$, $R_2$, $R_3$, $R_4$ and X have the given meanings, with bisulphite addition compounds of appropriate ketones (Hinsberg synthesis), or via an appropriate hydrazide (Brunner synthesis) or via an appropriate amide (Stollé synthesis);
and subsequently, if desired, converts compounds obtained of the formula I into other compounds of the formula I, as well as possibly converts the compounds into pharmacologically acceptable salts.

5. Process for the preparation of compounds of the formula I according to claim 4, in which $R_1$ and $R_2$ are the same and represent the methyl or ethyl group, $R_1$ and $R_2$ are different and signify hydrogen, the methyl, ethyl, isopropyl, cyclopentyl, cyano, acetyl, methoxycarbonyl, ethoxycarbonyl, aminocarbonyl and hydrazinocarbonyl group, $R_1$ and $R_2$ represent a spirocyclopentyl ring when $R_1$ and $R_2$, with the C-atom to which they are attached, form a cycloalkyl ring, $R_3$ signifies hydrogen, the methyl, ethyl, isopropyl, n-butyl, allyl, cyclohexyl, cyclopentenyl, cyano, ethoxycarbonyl or phenyl group, $R_4$ signifies the methyl, ethyl, isopropyl, trifluoromethyl, cyclopentyl, hydroxyl, cyano, acetyl, carboxyl, ethoxycarbonyl, aminocarbonyl, methylaminocarbonyl or dimethylaminocarbonyl group or $R_4$ the pyrrole, furan, thiophene, pyrazole, imidazole, thiazole, oxazole, triazole, tetrazole, thiadiazole, oxadiazole, pyridine, N-oxypyridine, pyrazine, N,N-dioxypyrazine, pyrimidine, N,N-dioxypyrimidine, pyridazine, oxazine, thiazine, triazine or tetrazine radical, whereby the stated residues can be substituted by methyl, ethyl, methoxy, ethoxy, methylmercapto, ethylmercapto and chlorine, or the phenyl radical of the general formula II, in which $R_5$ signifies a hydrogen, the methanesulphonyloxy, trifluoromethanesulphonyloxy, methanesulphonylamino, trifluoromethanesulphonylamino, methanesulphonylmethylamino, trifluoromethanesulphonylmethylamino, methylsulphenylmethyl, methylsulphinylmethyl, methylsulphonylmethyl, aminocarbonyl, aminosulphonyl, methylaminosulphonyl, dimethylaminosulphonyl, acetylamino, methylmercapto, methylsulphonyl, hydroxyl, methyl, methoxy, propargyloxy, cyanomethyloxy, methoxycarbonylmethyloxy, cyano, chloro, nitro, amino, dimethylamino, trifluoromethyl or the 1-imidazolyl radical, $R_6$ hydrogen, the methyl, methoxy, dimethylamino or chlorine group, $R_7$ is hydrogen or the methoxy group and X stands for an oxygen atom; their tautomers and their physiological acceptable salts.

6. Process for the preparation of compounds of the formula I according to claim 4, in which $R_1$ signifies the methyl or ethyl group, $R_2$ hydrogen, the methyl or ethyl group or together with $R_1$ the cyclopentyl ring, $R_3$ represents hydrogen, the methyl, ethyl or phenyl group, $R_4$ signifies the methyl, ethyl, hydroxyl, carboxyl, ethoxycarbonyl or aminocarbonyl group or the thiophene, pyridine or pyridazine radical or the phenyl group which can optionally be substituted by hydroxyl, methoxy, cyano, chlorine, methyl or 1-imidazolyl and X stands for an oxigen atom; their tautomers and their physiologically acceptable salts of inorganic and organic acids.

7. Use of compounds according to claim 1, 2 or 3 for the preparation of medicaments for the treatment of heart and/or circulatory diseases.

8. Medicaments containing a compound according to claim 1, 2 or 3, besides usual pharmaceutical carrier and/or adjuvant materials.

9. Compounds of the formula VI

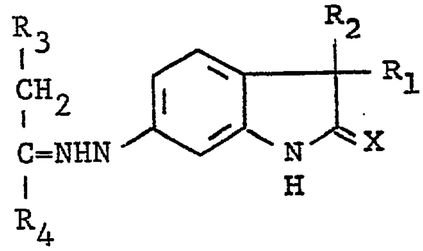

(VI)

in which $R_1$ signifies a hydrogen atom, a $C_1$–$C_6$-alkyl, $C_2$–$C_6$-alkenyl or $C_3$–$C_7$-cycloalkyl group, $R_2$ represents a hydrogen atom, a $C_1$–$C_6$-alkyl, $C_2$–$C_6$-alkenyl or cyano group, a carbonyl group substituted by a hydroxyl, alkyl, alkoxy, amino, alkylamino, dialkylamino or hydrazino group or together with $R_1$ represents a $C_3$–$C_7$-cycloalkylene group or $R_1$ and $R_2$ together form a $C_1$–$C_6$-alkylidene or $C_3$–$C_7$-cycloalkylidene group, whereby the above-mentioned alkyl or alkoxy moieties contain 1–6 C-atoms, $R_3$ signifies a hydrogen atom, an alkyl, alkenyl, cycloalkyl, cycloalkenyl, cyano, alkylcarbonyl, alkoxycarbonyl, carboxyl, aminocarbonyl, alkylaminocarbonyl, dialkylaminocarbonyl or aryl group, $R_4$ signifies a hydrogen atom, a $C_1$–$C_7$-alkyl, trihalogenomethyl, $C_3$–$C_7$-cycloalkyl, hydroxyl, cyano, carboxyl, alkoxycarbonyl, alkylcarbonyl, aminocarbonyl, alkylaminocarbonyl or dialkylaminocarbonyl group, whereby the above-mentioned alkyl or alkoxy moieties contain 1–7 C-atoms, or a heterocyclic five-membered ring with 1–4 heteroatoms or a heterocyclic six-membered ring with 1–5 heteroatoms, whereby the heteroatoms of the above-mentioned five- and six-membered rings can be the same or different and signify nitrogen, oxygen or sulphur and on one or more nitrogen atoms can optionally carry an oxygen atom and the above-mentioned five- and six-membered rings can optionally be substituted by one or more $C_1$–$C_6$-alkyl, $C_1$–$C_6$-alkoxy, $C_1$–$C_6$-alkylmercapto, hydroxyl, nitro, amino, halogen or cyano groups, or a phenyl ring of the general formula II

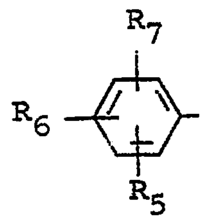

(II)

whereby $R_5$, $R_6$, $R_7$ can be the same or different and in each case can be hydrogen, an alkanesulphonyloxy, trifluoromethanesulphonyloxy, alkanesulphonylamino, trifluoromethanesulphonylamino, N-alkyl-alkanesulphonylamino, N-alkyl-trifluoromethanesulphonylamino, alkylsulphenylmethyl, alkylsulphinylmethyl or alkylsulphonylmethyl group, a carbonyl group substituted by a hydroxyl, alkoxy, amino, alkylamino or dialkylamino group, a sulphonyl group substituted by an amino, alkylamino, dialkylamino, morpholino, pyrrolidino, piperidino or hexamethyleneimino group, an alkylcarbonylamino, aminocarbonylamino or alkylaminocarbonylamino radical, an alkylmercapto, alkylsulphinyl or alkylsulphonyl group, a nitro, halogen, amino, hydroxyl, alkyl, alkoxy, alkenyloxy, alkynyloxy, cyanoalkyloxy, carboxyalkyloxy, alkoxycarbonylalkyloxy, dialkylamino, 1-imidazolyl, trifluoromethyl or cyano group, whereby the above-mentioned alkyl, alkenyl or alkoxy groups contain 1–5 C-atoms, and X represents an oxygen or sulphur atom.

10. Process for the preparation of compounds of the formula VI

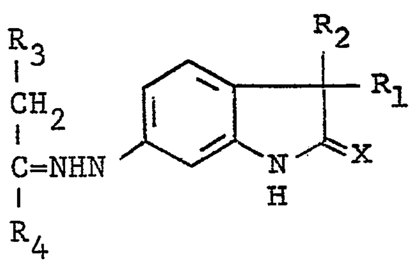

(VI)

in which $R_1$ signifies a hydrogen atom, a $C_1$–$C_6$-alkyl, $C_2$–$C_6$-alkenyl or a $C_3$–$C_7$-cycloalkyl group, $R_2$ represents a hydrogen atom, a $C_1$–$C_6$-alkyl, $C_2$–$C_6$-alkenyl or cyano group, a carbonyl group substituted by a hydroxyl, alkyl, alkoxy, amino, alkylamino, dialkylamino, or hydrazino group or together with $R_1$ represents a $C_3$–$C_7$-cycloalkylene group or $R_1$ and $R_2$ together form a $C_1$–$C_6$-alkylidene or $C_3$–$C_7$-cycloalkylidene group, whereby the above-mentioned alkyl or alkoxy moieties contain 1–6 C-atoms, $R_3$ signifies a hydrogen atom, a $C_1$–$C_7$-alkyl, $C_2$–$C_7$-alkenyl, $C_3$–$C_7$-cycloalkyl, $C_3$–$C_7$-cycloalkenyl, cyano, alkylcarbonyl, alkoxycarbonyl, carboxyl, aminocarbonyl, alkylaminocarbonyl, dialkylaminocarbonyl or aryl group, whereby the above-mentioned alkyl or alkoxy moieties contain 1–7 C-atoms, $R_4$ signifies a hydrogen atom, a $C_1$–$C_7$-alkyl, trihalogenomethyl, $C_3$–$C_7$-cycloalkyl, hydroxyl, cyano, carboxyl, alkoxycarbonyl, alkylcarbonyl, aminocarbonyl, alkylaminocarbonyl or dialkylaminocarbonyl group, whereby the above-mentioned alkyl or alkoxy moieties contain 1–7 C-atoms, or a heterocyclic five-membered ring with 1–4 heteroatoms or a heterocyclic six-membered ring with 1–5 heteroatoms, whereby the heteroatoms of the above-mentioned five- and six-membered rings can be the same or different and signify nitrogen, oxygen or sulphur and on one or more nitrogen atoms can optionally carry an oxygen atom and the above-mentioned five- and six-membered rings can optionally be substituted by one or more $C_1$–$C_6$-alkyl, $C_1$–$C_6$-alkyl, $C_1$–$C_6$-alkoxy, $C_1$–$C_6$-alkylmercapto, hydroxyl, nitro, amino, halogen or cyano groups, or a phenyl ring of the general formula II

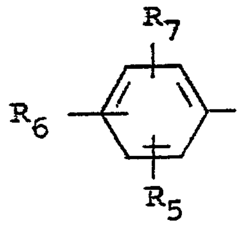

(II)

whereby $R_5$, $R_6$, $R_7$ can be the same or different and in each case can be hydrogen, an alkanesulphonyloxy, trifluoromethanesulphonyloxy, alkanesulphonylamino, trifluoromethanesulphonylamino, N-alkyl-alkanesulphonylamino, N-alkyl-trifluoromethanesulphonylamino, alkylsulphenylmethyl, alkylsulphinylmethyl or alkylsulphonylmethyl group, a carbonyl group substituted by a hydroxyl, alkoxy, amino, alkylamino or dialkylamino group, a sulphonyl group substituted by amino, alkylamino, dialkylamino, morpholino, pyrrolidino, piperidino or hexamethyleneimino group, an alkylcarbonylamino, aminocarbonylamino or alkylaminocarbonylamino group, an alkylmercapto, alkylsulphinyl or alkylsulphonyl group, a nitro, halogen, amino, hydroxyl, alkyl, alkoxy, alkenyloxy, alkynyloxy, cyanoalkyloxy, carboxyalkyloxy, alkoxycarbonylalkyloxy, dialkylamino, 1-imidazolyl, trifluoromethyl or cyano group, whereby the above-mentioned alkyl, alkenyl or alkoxy moieties contain 1–5 C-atoms, and X represents an oxygen or sulphur atom; characterised in that, by per se known methods, amines of the formula III

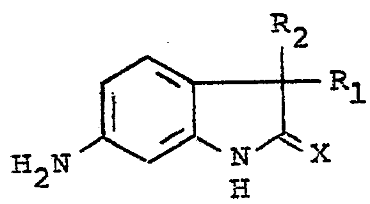

(III)

in which $R_1$, $R_2$ and X have the given meanings, are
A) diazotised, subsequently either
a) reduced to the hydrazines of the formula IV

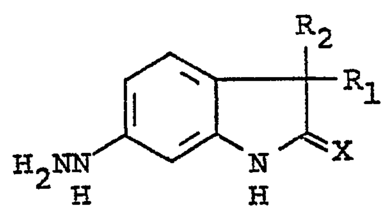

(IV)

in which $R_1$, $R_2$ and X have the given meanings, and reacted with compounds of the formula V

$R_4COCH_2R_3$ (V)

in which $R_3$ and $R_4$ have the given meanings; or
b) reacted in a Japp-Klingemann reaction with a compound of the formula VII

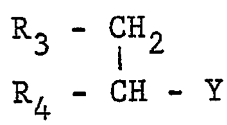

(VII)

in which $R_3$ and $R_4$ have the given meanings and Y represents an activating residue, and subsequently saponified; or
B) reacted with halogenoacetic acid esters X

$Z–CH_2–COOEt$ (X)

in which Z signifies halogen, subsequently saponified and nitrosated to the compounds VIII

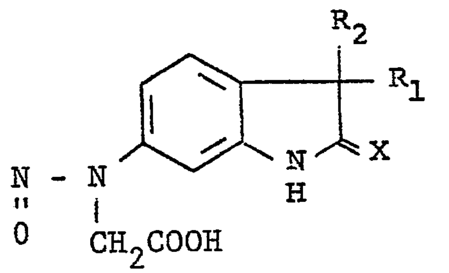

(VIII)

in which $R_1$, $R_2$ and X have the given meaning, these reacted with water-removing agents to the sydnones IX

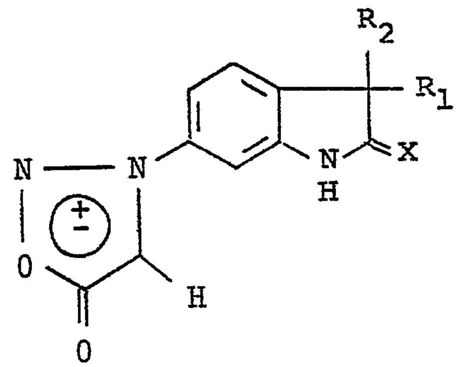

(IX)

which are converted with the ketones V

$R_4COCH_2R_3$ (V)

in which $R_3$ and $R_4$ have the above-given meanings, into compounds of the formula VI.

**Revendications**

1. Composé de formule I

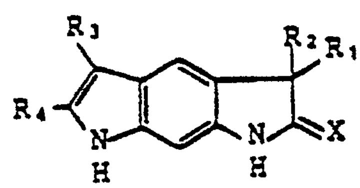

(I),

où

$R_1$ représente un atome d'hydrogène, un groupe alkyle en $C_1$–$C_6$, un groupe alcényle en $C_2$–$C_6$, ou un groupe cycloalkyle en $C_3$–$C_7$,

$R_2$ représente un atome d'hydrogène, un groupe alkyle en $C_1$–$C_6$, un groupe alcényle en $C_2$–$C_6$, ou un groupe cyano,

un groupe carbonyle substitué par un groupe hydroxy, alkyle, alcoxy, amino, alkylamino, dialkylamino ou hydrazino, ou en commun avec $R_1$, un groupe cycloalkylène en $C_3$–$C_7$, ou $R_1$ et $R_2$ représentent conjointement un groupe alkylidène en $C_1$–$C_6$ ou un groupe cycloalkylidène en $C_3$–$C_7$, les parties alkyle ou alcoxy mentionnées comportant 1–6 atomes de carbone,

$R_3$ représente un atome d'hydrogène, un groupe alkyle en $C_1$–$C_7$, un groupe alcényle en $C_2$–$C_7$, ou un groupe cycloalkyle en $C_3$–$C_7$, un groupe cycloalcényle en $C_3$–$C_7$, un groupe cyano, alkylcarbonyle, alkoxycarbonyle, carboxy, aminocarbonyle, alkylaminocarbonyle, dialkylaminocarbonyle ou aryle, les parties alkyle ou alcoxy mentionnées comportant 1–7 atomes de carbone,

$R_4$ représente un atome d'hydrogène, un groupe alkyle en $C_1$–$C_7$, un groupe trihalogénométhyle, ou un groupe cycloalkyle en $C_3$–$C_7$, un groupe hydroxy, cyano, carboxy, alcoxycarbonyle, alkylcarbonyle, alkylcarbonyle, aminocarbonyle, alkylaminocarbonyle ou dialkylaminocarbonyle, les parties alkyle ou alcoxy mentionnées comportant 1–7 atomes de carbone, ou

un hétérocycle à cinq chaînons avec 1–4 hétéroatomes, ou un hétérocycle à six chaînons avec 1–5 hétéroatomes, les hétéroatomes des cycles à cinq ou six chaînons mentionnés pouvant être identiques ou différents, et représentent l'azote, l'oxygène ou le soufre, et peuvent éventuellement porter un atome d'oxygène sur un ou plusieurs atomes d'azote, et les cycles à cinq et six chaînons mentionnées pouvant

être éventuellement substitués par un ou plusieurs groupes alkyle en $C_1$–$C_6$, alcoxy en $C_1$–$C_6$, alkylmercapto en $C_1$–$C_6$, hydroxy, nitro, amino, halogène ou cyano,
ou un cycle phényle de formule générale II

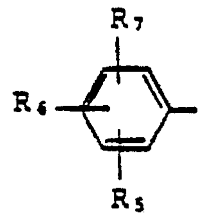

(II),

où $R_5$, $R_6$, $R_7$ peuvent être identiques ou différents et peuvent représenter chacun l'hydrogène, un groupe alcanesulfonyloxy, trifluorométhanesulfonyloxy, alcanosulfonylamino, trifluorométhanesulfonylamino, N-alkylalcanesulfonylamino, N-alkyl-trifluorométhanesulfonylamino, alkylsulfénylméthyle, alkylsulfinylméthyle ou alkylsulfonylméthyle, un groupe carbonyle substitué par un groupe hydroxy, alcoxy, amino, alkylamino ou dialkylamino, un groupe sulfonyle substitué par un groupe amino, alkylamino, dialkylamino, morpholino, pyrrolidino, pipéridino ou hexaméthylène-imino, un groupe alkylcarbonylamino, aminocarbonylamino ou alkylaminocarbonylamino, un groupe alkylmercapto, alkylsulfinyle ou alkylsulfonyle, un groupe nitro, halogèno, amino, hydroxy, alkyle, alcoxy, alcényloxy, alcinyloxy, cyanalkyloxy, carboxyalkyloxy, alcoxycarbonylalkyloxy, dialkylamino, 1-imidazolyle, trifluorométhyle ou cyano, les groupes alkyle, alcényle ou alcoxy mentionnés comportant 1 à 5 atomes de C,

X représente un atome d'oxygène ou de soufre,

leurs tautomères et leurs sels d'acides minéraux et organiques physiologiquement acceptables.

2. Composés de formule I selon la revendication 1, où

$R_1$ et $R_2$ sont identiques et représentent un groupe méthyle ou éthyle,

$R_1$ et $R_2$ sont différents et représentent l'hydrogène, le groupe méthyle, éthyle, isopropyle, cyclopentyle, cyano, acétyle, méthoxycarbonyle, éthoxycarbonyle, aminocarbonyle et hydrazinocarbonyle,

$R_1$ et $R_2$ représentent un cycle spyrocyclopentyle, lorsque $R_1$ et $R_2$ forment, conjointement avec l'atome de C auquel ils sont liés, un cycle cycloalkyle,

$R_3$ représente l'hydrogène, le groupe méthyle, éthyle, isopropyle, n-butyle, allyle, cyclohexyle, cyclopentènyle, cyano, éthoxycarbonyle ou phényle,

$R_4$ représente le groupe méthyle, éthyle, isopropyle, trifluorométhyle, cyclopentyle, hydroxy, cyano, acétyle, carboxy, éthoxycarbonyle, aminocarbonyle, méthylaminocarbonyle ou diméthylamino-carbonyle,

ou

$R_4$ représente le groupe pyrrole, furane, thiophène, pyrazole, imidazole, thiazole, oxazole, triazole, tétrazole, thiadiazole, oxadiazole, pyridine, N-oxypyridine, pyrazine, N,N-dioxy-pyrazine, pyrimidine, N,N-dioxy-pyrimidine, pyridazine, oxazine, thiazine, triazine ou tétrazine, les groupes mentionnés pouvant être substitués par des groupes méthyle, éthyle, méthoxy, éthoxy, méthylmercapto, éthylmercapto et chloro,

ou le groupe phényle de formule générale II où

$R_5$ représente l'hydrogène, le groupe méthanesulfonyloxy, trifluorométhanesulfonyloxy, méthanesulfonylamino, trifluorométhanesulfonylamino, méthanesulfonylméthylamino, trifluorométhanesulfonylméthylamino, méthylsulfènylméthyle, méthylsulfinylméthyle, méthylsulfonylméthyle, aminocarbonyle, aminosulfonyle, méthylaminosulfonyle, diméthylaminosulfonyle, acétylamino, méthylmercapto, méthylsulfonyle, hydroxy, méthyle, méthoxy, propargyloxy, cyanométhyloxy, méthoxycarbonyleméthyloxy, cyano, chloro, nitro, amino, diméthylamino, trifluorométhyle ou le groupe 1-imidazolyle,

$R_6$ représente l'hydrogène, le groupe méthyle, méthoxy, diméthylamino ou chloro,

$R_7$ représente l'hydrogène ou le groupe méthoxy, et

X représente un atome d'oxygène,

leurs tautomères et leurs sels d'acides minéraux et organiques physiologiquement acceptables.

3. Composés de formule I selon la revendication 1 ou 2, où

$R_1$ représente le groupe méthyle ou éthyle,

$R_2$ représente l'hydrogène, le groupe méthyle ou éthyle ou conjointement avec $R_1$, le cycle cyclopentyle,

$R_3$ représente l'hydrogène, le groupe méthyle, éthyle ou phényle,

$R_4$ représente le groupe méthyle, éthyle, hydroxy, carboxy, éthoxycarbonyle ou aminocarbonyle, ou le groupe thiophène, pyridine ou pyridazine, ou le groupe phényle, qui peut être éventuellement substitué par le groupe hydroxy, méthoxy, cyano, chloro, méthyle ou 1-imidazolyle, et

X représente un atome d'oxygène,

leurs tautomères et leurs sels d'acides minéraux ou organiques physiologiquement acceptables.

4. Procédé pour la préparation de composés de formule I

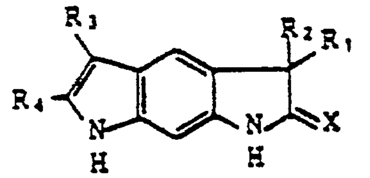

(I),

où

$R_1$ représente un atome d'hydrogène, un groupe alkyle en $C_1$–$C_6$, un groupe alcényle en $C_2$–$C_6$, ou un groupe cycloalkyle en $C_3$–$C_7$,

$R_2$ représente un atome d'hydrogène, un groupe alkyle en $C_1$–$C_6$, un groupe alcényle en $C_2$–$C_6$, ou un groupe cyano,

un groupe carbonyle substitué par un groupe hydroxy, alkyle, alcoxy, amino, alkylamino, dialkylamino ou hydrazino, ou en commun avec $R_1$ un groupe cycloalkylène en $C_3$–$C_7$, ou $R_1$ et $R_2$ représentent conjointement un groupe alkylidène en $C_1$–$C_6$, ou un groupe cycloalkylidène en $C_3$–$C_7$, les parties alkyle ou alcoxy mentionnées comportant 1–6 atomes de carbone,

$R_3$ représente un atome d'hydrogène, un groupe alkyle en $C_1$–$C_7$, un groupe alcényle en $C_2$–$C_7$, ou un groupe cycloalkyle en $C_3$–$C_7$, un groupe cycloalcényle en $C_3$–$C_7$, un groupe cyano, alkylcarbonyle, alcoxycarbonyle, carboxy, aminocarbonyle, alkylaminocarbonyle, dialkylaminocarbonyle ou aryle, les parties alkyle ou alcoxy mentionnées comportant 1–7 atomes de carbone,

$R_4$ représente un atome d'hydrogène, un groupe alkyle en $C_1$–$C_7$, un groupe trihalogénométhyle, ou un groupe cycloalkyle en $C_3$–$C_7$, un groupe hydroxy, cyano, carboxy, alcoxycarbonyle, alkylcarbonyle, aminocarbonyle, alkylaminocarbonyle ou dialkylaminocarbonyle, les parties alkyle ou alkoxy mentionnées comportant 1–7 atomes de carbone, ou

un hétérocycle à cinq chaînons avec 1–4 hétéroatomes, ou un hétérocycle à six chaînons avec 1–5 hétéroatomes, les hétéroatomes des cycles à cinq ou six chaînons mentionnés pouvant être identiques ou différents, et représentent l'azote, l'oxygène ou le soufre, et peuvent éventuellement porter un atome d'oxygène sur un ou plusieurs atomes d'azote, et les cycles à cinq ou six chaînons mentionnés pouvant être éventuellement substitués par un ou plusieurs groupes alkyle en $C_1$–$C_6$, alcoxy en $C_1$–$C_6$, alkylmercapto en $C_1$–$C_6$, hydroxy, nitro, amino, halogèno ou cyano,

ou un cycle phényle de formule générale II

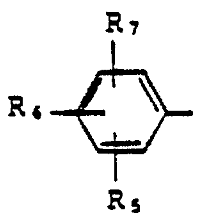

(II),

où $R_5$, $R_6$, $R_7$ peuvent être identiques ou différents et peuvent représenter chacun l'hydrogène, un groupe alcanesulfonyloxy, trifluorométhanesulfonyloxy, alcanesulfonylamino, trifluorométhanesulfonylamino, N-alkyl-alcanesulfonylamino, N-alkyl-trifluorométhanesulfonylamino, alkylsulfénylméthyle, alkylsulfinylméthyle ou alkylsulfonylméthyle, un groupe carbonyle substitué par un groupe hydroxy, alcoxy, amino, alkylamino ou dialkylamino, un groupe sulfonyle substitué par un groupe amino, alkylamino, dialkylamino, morpholino, pyrrolidino, pipéridino ou hexaméthylène-imino, un groupe alkylcarbonylamino, aminocarbonylamino ou alkylaminocarbonylamino, un groupe alkylmercapto, alkylsulfinyle ou alkylsulfonyle, un groupe nitro, halogèno, amino, hydroxy, alkyle, alcoxy, alcényloxy, alcinyloxy, cyanalkyloxy, carboxyalkyloxy, alcoxycarbonalkyloxy, dialkylamino, 1-imidazolyle, trifluorométhyle ou cyano, les groupes alkyle, alcényle et alcoxy mentionnés comportant 1 à 5 atomes de C,

X représente un atome d'oxygène ou de soufre,

leurs tautomères et leurs sels physiologiquement acceptables,

caractérisé en ce que, de manière connue en soi, on cyclise

a) un composé de formule VI

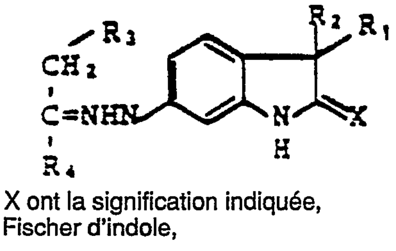

(VI),

où $R_1$, $R_2$, $R_3$, $R_4$ et X ont la signification indiquée,

selon une synthèse Fischer d'indole,

ou
b) un composé de formule III

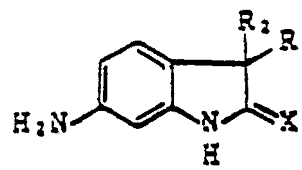

(III)

ou de formule XI

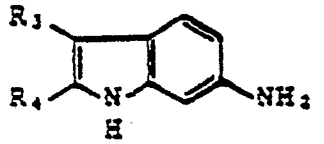

(XI),

où $R_1$, $R_2$, $R_3$, $R_4$ et X ont la signification indiquée,
avec des composés d'addition de bisulfite de cétones appropriés (synthèse de Hinsberg)
ou par l'intermédiaire d'un hydrazide approprié (synthèse de Brunner)
ou par l'intermédiaire d'un amide approprié (synthèse de Stollé)
et ensuite, on transforme éventuellement les composés de formule I obtenus en d'autres composés de formule I, et éventuellement, on transforme les composés en leurs sels physiologiquement acceptables.

5. Procédé pour la préparation de composés de formule I selon la revendication 4,
où
$R_1$ et $R_2$ sont identiques et représentent le groupe méthyle ou éthyle,
$R_1$ et $R_2$ sont différents et représentent l'hydrogène, le groupe méthyle, éthyle, isopropyle, cyclopentyle, cyano, acétyle, méthoxycarbonyle, éthoxycarbonyle, aminocarbonyle et hydrazinocarbonyle,
$R_1$ et $R_2$ représentent un cycle spyrocyclopentyle, lorsque $R_1$ et $R_2$ forment, conjointement avec l'atome de C auquel ils sont liés, un cycle cycloalkyle,
$R_3$ représente l'hydrogène, le groupe méthyle, éthyle, isopropyle, n-butyle, allyle, cyclohexyle, cyclopentènyle, cyano, éthoxycarbonyle ou phényle,
$R_4$ représente le groupe méthyle, éthyle, isopropyle, trifluorométhyle, cyclopentyle, hydroxy, cyano, acétyle, carboxy, éthoxycarbonyle, aminocarbonyle, méthylaminocarbonyle ou diméthylaminocarbonyle ou diméthylamino-carbonyle,
ou
$R_4$ représente le groupe pyrrole, furane, thiophène, pyrazole, imidazole, thiazole, oxazole, triazole, tétrazole, thiadiazole, oxadiazole, pyridine, N-oxy-pyridine, pyrazine, N,N-dioxy-pyrazine, pyrimidine, N,N-dioxy-pyrimidine, pyridazine, oxazine, thiazine, triazine ou tétrazine, les groupes mentionnés pouvant être substitués par des groupes méthyle, éthyle, méthoxy, éthoxy, méthylmercapto, éthylmercapto et chloro,
ou le groupe phényle de formule générale II où
$R_5$ représente l'hydrogène, le groupe méthanesulfonyloxy, trifluorométhanesulfonyloxy, méthanesulfonylamino, trifluorométhanesulfonylamino, méthanesulfonylméthylamino, trifluorométhanesulfonylméthylamino, méthylsulfènylméthyle, méthylsulfinylméthyle, méthylsulfonylméthyle, aminocarbonyle, aminosulfonyle, méthylaminosulfonyle, diméthylaminosulfonyle, acétylamino, méthylmercapto, méthylsulfonyle, hydroxy, méthyle, méthoxy, propargyloxy, cyanométhyloxy, méthoxycarbonyleméthyloxy, cyano, chloro, nitro, amino, diméthylamino, trifluorométhyle ou le groupe 1-imidazolyle,
$R_6$ représente l'hydrogène, le groupe méthyle, méthoxy, diméthylamino ou chloro,
$R_7$ représente l'hydrogène ou le groupe méthoxy, et
X représente un atome d'oxygène,
leurs tautomères et leurs sels physiologiquement acceptables.

6. Procédé pour la préparation de composés de formule I selon la revendication 4,
où
$R_1$ représente le groupe méthyle ou éthyle,
$R_2$ représente l'hydrogène, le groupe méthyle ou éthyle, ou forme, conjointement avec $R_1$, un cycle cyclopentyle,
$R_3$ représente l'hydrogène, le groupe méthyle, éthyle ou phényle,
$R_4$ représente le groupe méthyle, éthyle, hydroxy, carboxy, éthoxycarbonyle ou aminocarbonyle, ou le groupe thiophène, pyridine ou pyridazine ou le groupe phényle, qui peut éventuellement être substitué par un groupe hydroxy, méthoxy, cyano, chloro, méthyle ou 1-imidazolyle, et
X représente un atome d'oxygène,

leur tautomères et leurs sels d'acides minéraux ou organiques physiologiquement acceptables.

7. Utilisation des composés selon les revendications 1, 2 ou 3, pour la préparation de médicaments pour le traitement de maladies cardiaques et/ou cardiovasculaires.

8. Médicament contenant un composé selon les revendications 1, 2 ou 3, en plus de véhicules et/ou adjuvants pharmaceutiques connus.

9. Composés de formule VI

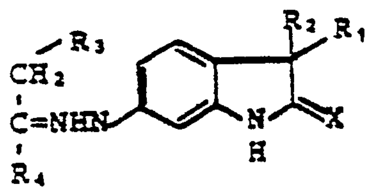
(VI),

où

$R_1$ représente un atome d'hydrogène, un groupe alkyle en $C_1$–$C_6$, un groupe alcényle en $C_2$–$C_6$, ou un groupe cycloalkyle en $C_3$–$C_7$,

$R_2$ représente un atome d'hydrogène, un groupe alkyle en $C_1$–$C_6$, un groupe alcényle en $C_2$–$C_6$, ou un groupe cyano,

un groupe carbonyle substitué par un groupe hydroxy, alkyle, alcoxy, amino, alkylamino, dialkylamino ou hydrazino, ou en commun avec $R_1$ un groupe cycloalkylène en $C_3$–$C_7$, ou $R_1$ et $R_2$ représentent conjointement un groupe alkylidène en $C_1$–$C_6$, ou un groupe cycloalkylidène en $C_3$–$C_7$, les parties alkyle ou alcoxy mentionnées comportant 1–6 atomes de carbone,

$R_3$ représente un atome d'hydrogène, un groupe alkyle, un groupe alcényle, ou un groupe cycloalkyle, un groupe cycloalcényle, un groupe cyano, alkylcarbonyle, alcoxycarbonyle, carboxy, aminocarbonyle, alkylaminocarbonyle, dialkylaminocarbonyle ou aryle,

$R_4$ représente un atome d'hydrogène, un groupe alkyle en $C_1$–$C_7$, un groupe trihalogénométhyle, ou un groupe cycloalkyle en $C_3$–$C_7$, un groupe hydroxy, cyano, carboxy, alcoxycarbonyle, alkylcarbonyle, aminocarbonyle, alkylaminocarbonyle ou dialkylaminocarbonyle, les parties alkyle ou alcoxy mentionnées comportant 1–7 atomes de carbone, ou

un hétérocycle à cinq chaînons avec 1–4 hétéroatomes, ou un hétérocycle à six chaînons avec 1–5 hétéroatomes, les hétéroatomes des cycles à cinq et six chaînons mentionnés pouvant être identiques ou différents, et représentent l'azote, l'oxygène ou le soufre, et peuvent éventuellement porter un atome d'oxygène sur un ou plusieurs atomes d'azote, et les cycles à cinq et six chaînons mentionnés pouvant être éventuellement substitués par un ou plusieurs groupes alkyle en $C_1$–$C_6$, alcoxy en $C_1$–$C_6$, alkylmercapto en $C_1$–$C_6$, hydroxy, nitro, amino, halogéno ou cyano,

ou un noyau phényle de formule générale II

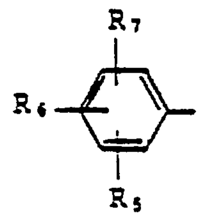
(II),

où $R_5$, $R_6$, $R_7$ peuvent être identiques ou différents et peuvent représenter chacun l'hydrogène, un groupe alcanesulfonyloxy, trifluorométhanesulfonyloxy, alcanesulfonylamino, trifluorométhanesulfonylamino, N-alkyl-alcanesulfonylamino, N-alkyl-trifluorométhanesulfonylamino, alkylsulfénylméthyle, alkylsulfinylméthyle ou alkylsulfonylméthyle, un groupe carbonyle substitué par un groupe hydroxy, alcoxy, amino, alkylamino ou dialkylamino, un groupe sulfonyle substitué par un groupe amino, alkylamino, dialkylamino, morpholino, pyrrolidino, pipéridino ou hexaméthylène-imino, un groupe alkylcarbonylamino, aminocarbonylamino ou alkylaminocarbonylamino, un groupe alkylmercapto, alkylsulfinyle ou alkylsulfonyle, un groupe nitro, halogèno, amino, hydroxy, alkyle, alcoxy, alcényloxy, alcinyloxy, cyanalkyloxy, carboxylalkyloxy, alcoxycarbonylalkyloxy, dialkylamino, 1-imidazolyle, trifluorométhyle ou cyano, les groupes alkyle, alcényle et alcoxy mentionnés comportant 1 à 5 atomes de C,

X représente un atome d'oxygène ou de soufre.

10. Procédé pour la préparation de composés de formule VI

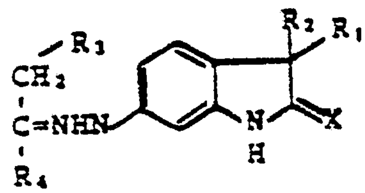

(VI),

où

$R_1$ représente un atome d'hydrogène, un groupe alkyle en $C_1$–$C_6$, un groupe alcényle en $C_2$–$C_6$, ou un groupe cycloalkyle en $C_3$–$C_7$,

$R_2$ représente un atome d'hydrogène, un groupe alkyle en $C_1$–$C_6$, un groupe alcényle en $C_2$–$C_6$, ou un groupe cyano,

un groupe carbonyle substitué par un groupe hydroxy, alkyle, alcoxy, amino, alkylamino, dialkylamino ou hydrazino, ou en commun avec $R_1$ un groupe cycloalkylène en $C_3$–$C_7$, où $R_1$ et $R_2$ représentent conjointement un groupe alkylidène en $C_1$–$C_6$, ou un groupe cycloalkylidène en $C_3$–$C_7$, les parties alkyle ou alcoxy mentionnées comportant 1–6 atomes de carbone, $R_3$ représente un atome d'hydrogène, un groupe alkyle en $C_1$–$C_7$, un groupe alcényle en $C_2$–$C_7$, ou un groupe cycloalkyle en $C_3$–$C_7$, un groupe cycloalcényle en $C_3$–$C_7$, un groupe cyano, alkylcarbonyle, alcoxycarbonyle, carboxy, aminocarbonyle, alkylaminocarbonyle, dialkylaminocarbonyle ou aryle, les parties alkyle ou alcoxy mentionnées comportant 1–7 atomes de carbone,

$R_4$ représente un atome d'hydrogène, un groupe alkyle en $C_1$–$C_7$, un groupe trihalogénométhyle, ou un groupe cycloalkyle en $C_3$–$C_7$, un groupe hydroxy, cyano, carboxy, alcoxycarbonyle, alkylcarbonyle, aminocarbonyle, alkylaminocarbonyle ou dialkylaminocarbonyle, les parties alkyle ou alcoxy mentionnées comportant 1–7 atomes de carbone, ou

un hétérocycle à cinq chaînons avec 1–4 hétéroatomes, ou un hétérocycle à six chaînons avec 1–5 hétéroatomes, les hétéroatomes des cycles à cinq ou six chaînons mentionnés pouvant être identiques ou différents, et représentent l'azote, l'oxygène ou le soufre, et peuvent éventuellement porter un atome d'oxygène sur un ou plusieurs atomes d'azote, et les cycles à cinq ou six chaînons mentionnés pouvant être éventuellement substitués par un ou plusieurs groupes alkyle en $C_1$–$C_6$, alcoxy en $C_1$–$C_6$, alkylmercapto en $C_1$–$C_6$, hydroxy, nitro, amino, halogène ou cyano,

ou un cycle phényle de formule générale II

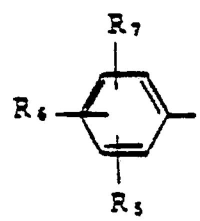

(II),

où $R_5$, $R_6$, $R_7$ peuvent être identiques ou différents et peuvent représenter chacun l'hydrogène, un groupe alcanesulfonyloxy, trifluorométhanesulfonyloxy, alcanesulfonylamino, trifluorométhanesulfonylamino, N-alkyl-alcanesulfonylamino, N-alkyl-trifluorométhanesulfonylamino, alkylsulfénylméthyle, alkylsulfinylméthyle ou alkylsulfonylméthyle, un groupe carbonyle substitué par un groupe hydroxy, alcoxy, amino, alkylamino ou dialkylamino, un groupe sulfonyle substitué par un groupe amino, alkylamino, dialkylamino, morpholino, pyrrolidino, pipéridino ou hexaméthylène-imino, un groupe alkylcarbonylamino, aminocarbonylamino ou alkylaminocarbonylamino, un groupe alkylmercapto, alkylsulfinyle ou alkylsulfonyle, un groupe nitro, halogèno, amino, hydroxy, alkyle, alcoxy, alcényloxy, alcinyloxy, cyanalkyloxy, carboxyalkyloxy, alcoxycarbonylalkyloxy, dialkylamino, 1-imidazolyle, trifluorométhyle ou cyano, les groupes alkyle, alcényle et alcoxy mentionnés comportant 1 à 5 atomes de C,

X représent un atome d'oxygène ou de soufre,

caractérisé en ce que, de manière connue en soi, l'amine de formule III

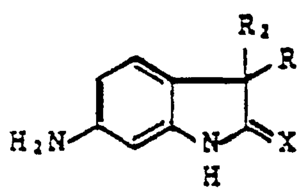

(III),

où $R_1$, $R_2$ et X ont la signification indiquée,

A) est diazotée, et ensuite, soit

a) réduite en hydrazino de formule IV

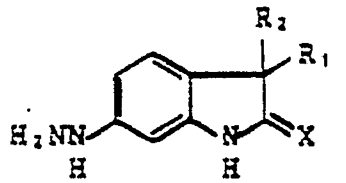

(IV),

où $R_1$, $R_2$ et X ont la signification indiquée,
et est amenée à réagir avec des composés de formule V

$R_4COCH_2R_3$ (V),

où $R_3$ et $R_4$ ont la signification indiquée,
soit
b) elle est amenée à réagir, selon la réaction de Japp-Klingemann, avec un composé de formule VII

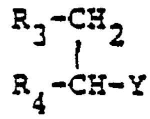

(VII),

où $R_3$ et $R_4$ ont la signification indiquée, et Y représente un groupe activant, et ensuite elle est saponifiée,
soit
B) elle est amenée à réagir avec des halogénoacétates X

$Z\text{–}CH_2\text{–}COOEt$ (X),

où Z représent un halogène, puis saponifiée et nitrosée en composé VIII

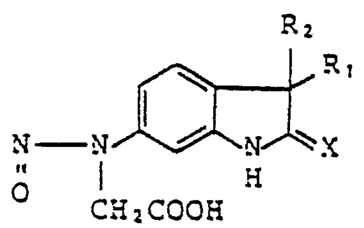

(VIII),

où $R_1$, $R_2$ et X ont la signification indiquée,
on fait réagir les composés VIII avec des réactifs déshydratants en sydnones IX

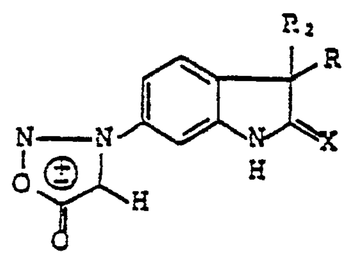

(IX),

qui sont transformés avec les cétones V,

$R_4COCH_2R_3$ (V),

où $R_3$ et $R_4$ ont la signification indiquée, en composés de formule VI.